(19) Europäisches Patentamt / European Patent Office / Office européen des brevets

(11) **EP 4 445 120 B1**

(12) **FASCICULE DE BREVET EUROPEEN**

(45) Date de publication et mention de la délivrance du brevet:
**22.07.2026 Bulletin 2026/30**

(21) Numéro de dépôt: **22847539.8**

(22) Date de dépôt: **09.12.2022**

(51) Classification Internationale des Brevets (IPC):
**G01N 21/64** $^{(2006.01)}$     **G01N 33/18** $^{(2006.01)}$
**C02F 3/00** $^{(2023.01)}$     **C02F 1/00** $^{(2023.01)}$

(52) Classification Coopérative des Brevets (CPC):
**C02F 1/008; G01N 21/64; G01N 33/1826;**
C02F 2209/006; C02F 2209/10; C02F 2209/11;
G01N 2021/6417

(86) Numéro de dépôt international:
**PCT/FR2022/052291**

(87) Numéro de publication internationale:
**WO 2023/105174 (15.06.2023 Gazette 2023/24)**

(54) **METHODE ET SYSTEME DE CONTROLE EN LIGNE DE L'ELIMINATION DE MICROPOLLUANTS DES EAUX USEES D'UNE UNITE DE TRAITEMENT**

VERFAHREN UND SYSTEM ZUR ON-LINE-REGULIERUNG DER ENTFERNUNG VON MIKROSCHADSTOFFEN AUS DEM ABWASSER EINER VERARBEITUNGSEINHEIT

METHOD AND SYSTEM FOR ON-LINE REGULATION OF THE REMOVAL OF MICROPOLLUTANTS FROM THE WASTE WATER OF A PROCESSING UNIT

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité: **10.12.2021 FR 2113252**

(43) Date de publication de la demande:
**16.10.2024 Bulletin 2024/42**

(73) Titulaire: **SUEZ International**
**92800 Puteaux (FR)**

(72) Inventeurs:
• **GUILLOSSOU, Ronan**
**93160 NOISY-LE-GRAND (FR)**
• **GONZALEZ OSPINA, Adriana**
**78360 MONTESSON (FR)**

(74) Mandataire: **Lavoix**
**2, place d'Estienne d'Orves**
**75441 Paris Cedex 09 (FR)**

(56) Documents cités:
**WO-A1-2014/186167**

• SGROI MASSIMILIANO ET AL: "Comparison of the new Cl2/O3/UV process with different ozone- and UV-based AOPs for wastewater treatment at pilot scale: Removal of pharmaceuticals and changes in fluorescing organic matter", SCIENCE OF THE TOTAL ENVIRONMENT, ELSEVIER, AMSTERDAM, NL, vol. 765, 6 October 2020 (2020-10-06), XP086492552, ISSN: 0048-9697, [retrieved on 20201006], DOI: 10.1016/J.SCITOTENV.2020.142720
• GUILLOSSOU RONAN ET AL: "Fluorescence excitation/emission matrices as a tool to monitor the removal of organic micropollutants from wastewater effluents by adsorption onto activated carbon", WATER RESEARCH, ELSEVIER, AMSTERDAM, NL, vol. 190, 14 December 2020 (2020-12-14), XP086459862, ISSN: 0043-1354, [retrieved on 20201214], DOI: 10.1016/J.WATRES.2020.116749

   

- **NIE JIANXIN ET AL: "Development of fluorescence surrogates to predict the ferrate(VI) oxidation of pharmaceuticals in wastewater effluents", WATER RESEARCH, ELSEVIER, AMSTERDAM, NL, vol. 185, 31 July 2020 (2020-07-31), XP086292206, ISSN: 0043-1354, [retrieved on 20200731], DOI: 10.1016/ J.WATRES.2020.116256**

# EP 4 445 120 B1

**Description**

## DOMAINE DE L'INVENTION

L'invention concerne le domaine du traitement de l'eau, et plus particulièrement le traitement des substances présentes à l'état de traces aussi appelées micropolluants. L'invention concerne en particulier une méthode de contrôle en ligne de l'élimination de ces micropolluants.

## ARRIERE-PLAN TECHNOLOGIQUE

Le terme « micropolluant » désigne un groupe de substances chimiques qui proviennent au moins en partie des activités humaines et qui ont des effets néfastes connus ou supposés même aux faibles concentrations (de ng/L à μg/L) auxquelles elles sont présentes dans l'environnement. Parmi les 100 000 substances chimiques référencées en Europe, certaines reconnues comme "micropolluants" sont réglementées et d'autres non. Elles peuvent être regroupées par familles chimiques, usages, effets ou statut réglementaire, et un micropolluant peut ainsi appartenir à plusieurs groupes. Les effluents industriels, les rejets urbains, l'agriculture et les produits agricoles sont des sources qui ont été identifiées et étudiées depuis les années 1970. Plus récemment, des sources de micropolluants liés aux émissions domestiques (produits pharmaceutiques, cosmétiques, détergents, pesticides, solvants, tensioactifs, plastifiants, retardateurs de flamme, etc.) ont été identifiées et sont désormais au cœur des problèmes associés aux micropolluants. Ainsi, depuis quelques années, les eaux traitées à potabiliser mais aussi les eaux traitées déversées dans le milieu naturel, doivent répondre à des exigences règlementaires de plus en plus strictes et la teneur en micropolluants doit être contrôlée afin de ne pas dépasser certains seuils.

Les technologies utilisées pour éliminer ces micropolluants des eaux sont typiquement l'adsorption, par exemple sur charbon actif en poudre ou en grain, et l'ozonation. La quantité de réactif (adsorbant ou ozone) utilisée et/ou consommée dépend alors de la concentration de l'eau à traiter en micropolluants. Un contrôle de cette quantité de réactif est alors préférable afin de réduire les coûts de traitement tout en assurant l'abattement souhaité des micropolluants.

Il existe ainsi des méthodes permettant de contrôler et/ou de suivre l'abattement des micropolluants, et, in fine, d'optimiser les concentrations en réactifs nécessaires à l'élimination de ces micropolluants.

Ainsi, le document FR3009789A1 propose une méthode de contrôle de l'abattement des micropolluants organiques dans les eaux usées par adsorption sur charbon actif dans laquelle on réalise régulièrement des mesures d'absorbance UV (ultraviolet) des eaux à traiter avant et après traitement. Ces mesures d'absorbance UV permettent de calculer le pourcentage d'abattement des micropolluants organiques et de réguler la concentration en charbon actif injectée dans les eaux à traiter en fonction de ce pourcentage calculé. Cette méthode utilise une corrélation entre le pourcentage de diminution de l'absorbance (déterminé à partir des absorbances mesurées avant et après traitement) et l'abattement qui n'est toutefois pas toujours assez robuste pour obtenir l'efficacité souhaitée d'élimination des micropolluants. Il est par ailleurs difficile de corréler les mesures d'absorbance UV à l'élimination d'un micropolluant particulier. Enfin, les mesures d'absorbance UV peuvent être perturbées par la présence de certaines molécules organiques, notamment présentes dans les eaux de provenance autre que domestique qui sont collectées avec les eaux usées, de sorte que la quantité de réactif ajoutée n'est pas contrôlée de manière précise.

Le document EP3348995B1 décrit un procédé de dosage d'ozone ou de charbon actif en fonction des besoins d'une installation de traitement des eaux usées qui utilise également des mesures d'absorption UV avant et après traitement. Ce procédé souffre ainsi des mêmes inconvénients que le procédé décrit dans le précédent document.

On connait également du document EP3174833A1 un procédé de contrôle d'une installation de traitement d'eau par ozonation dans lequel on effectue des mesures d'ensembles de paramètres au moyen de sondes de fluorescence ou de sondes UV-visible en entrée et en sortie d'un traitement par ozonation et en sortie d'une filtration biologique réalisée après le traitement par ozonation. Ces paramètres permettent de mesurer une première et une deuxième concentration en micropolluants avant et après le traitement d'ozonation, utilisées pour contrôler ce dernier afin de maintenir un abattement en micropolluants dans un intervalle prédéfini, et une troisième concentration en micropolluants après la filtration biologique utilisée pour augmenter la quantité d'ozone lorsque cette troisième concentration est inférieure à un seuil. Les paramètres utilisés sont choisis parmi le COT (carbone organique total), la DCO (demande chimique en oxygène) et le COD (carbone organique dissous). Ce document ne détaille que la mesure par UV visible.

Le document JP2002166265A décrit un système de contrôle de traitement de l'eau au moyen d'analyseurs de fluorescence permettant en particulier de réduire le potentiel de formation de trihalométhane résultant d'un traitement au chlore. Le système permet notamment d'ajouter une quantité de charbon actif permettant d'éliminer les précurseurs de trihalométhane. A cet effet, des mesures de fluorescence sont réalisées en amont et en aval de l'ajout de charbon actif. Le système permet également de contrôler une quantité d'ozone introduite dans des réacteurs de traitement à l'ozone. Des analyseurs de fluorescence mesurent des intensités de fluorescence relative de l'eau par calcul d'un spectre de fluorescence à une longueur d'onde d'excitation de 345nm et par calcul d'un spectre d'excitation correspondant à un spectre de fluorescence à 425nm. Le procédé utilise des corrélations entre cette intensité de fluorescence relative et le potentiel de formation de trihalométhane, entre le taux d'injection de charbon actif et un taux résiduel de l'intensité de fluorescence relative. Ce document n'enseigne pas l'utilisation de mesures de fluorescence pour déterminer un taux d'abattement en micropolluants.

Les procédés existants ne permettent pas d'estimer une efficacité d'élimination moyenne de chaque micropolluant parmi un groupe de micropolluants, même en présence de molécules organiques susceptibles de perturber la mesure, ce qui peut permettre d'améliorer le suivi du traitement et de minimiser la quantité de réactif à ajouter. En outre, les liens entre la fluorescence des matières organiques dissoutes et l'élimination des micropolluants sont encore mal compris, rendant difficile l'utilisation de la spectroscopie par fluorescence.

Il a été montré que le taux de diminution de la fluorescence totale (notée TF : somme des intensités de fluorescence intégrées au niveau régional) peut être corrélé au pourcentage d'abattement d'un micropolluant (Park et al, Chemosphere193(2018) 530-537). La mesure de l'intensité de fluorescence totale prend toutefois du temps (au moins 15 minutes), de sorte qu'il est impossible d'utiliser cette mesure pour un suivi de l'abattement en temps réel. En outre, la fluorescence totale correspond à l'ensemble de la matière organique dissoute fluorescente, de sorte qu'il est difficile, voire impossible, de corréler cette fluorescence totale à des fractions spécifiques de la matière organique dissoute qui interagissent en solution avec les micropolluants et/ou ont des mécanismes d'oxydation et d'adsorption similaires aux micropolluants.

WO 2014/186167 A1 décrit une corrélation entre d'une part un signal de fluorescence mesurée dans une eau et d'autre part une demande chimique en oxygène, une demande biochimique en oxygène, un ratio de ces deux demandes ou un carbone organique total. Sgroi Massimiliano et al., "Comparison of the new Cl2/O3/UV process with different ozone- and UV-based AOPs for wastewater treatment at pilot scale: Removal of pharmaceuticals and changes in fluorescing organic matter", SCIENCE OF THE TOTAL ENVIRONMENT, ELSEVIER, AMSTERDAM, NL, vol. 765, 6 octobre 2020, ISSN: 0048-9697, DOI: 10.1016/J.SCITOTENV.2020.142720, ainsi que Nie Jianxin et al., "Development of fluorescence surrogates to predict the ferrate (VI) oxidation of pharmaceuticals in wastewater effluents", WATER RESEARCH, ELSEVIER, AMSTERDAM, NL, vol. 185, 31 juillet 2020, ISSN: 0043-1354, DOI: 10.1016/J.WATRES.2020.116256, décrivent le suivi de l'élimination de micropolluants au moyen d'une analyse PARAFAC.

Guillossou et al., "Fluorescence excitation/emission matrices as a tool to monitor the removal of organic micropollutants from wastewater effluents by adsorption onto activated carbon", WATER RESEARCH, ELSEVIER, AMSTERDAM, NL, vol. 190, 14 décembre 2020, ISSN: 0043-1354, DOI: 10.1016/J.WATRES.2020.116749 ; ont par ailleurs montré que des corrélations peuvent été établies entre des indicateurs spectroscopiques par fluorescence et l'efficacité d'élimination de micropolluants. Ces corrélations sont en particulier plus robustes que les corrélations obtenues par la mesure de l'absorbance UV.

Il existe cependant un besoin pour une méthode de contrôle permettant d'améliorer le suivi de l'élimination des micropolluants.

Par ailleurs, les unités de traitement par adsorption comprennent typiquement un réacteur ou plus contenant chacun au moins un réactif qui est un adsorbant. L'efficacité de ces unités de traitement dépend de la masse de réactif disponible pour absorber le ou les polluants. L'adsorption des polluants met toutefois en œuvre des mécanismes relativement complexes qui rendent le suivi de l'adsorption difficile. Ainsi, actuellement, les stratégies de remplacement de l'adsorbant dans une unité de traitement sont souvent définies à l'avance, éventuellement via un suivi de la DCO. Ces stratégies consistent à arrêter l'un des réacteurs de l'unité de traitement pour remplacer intégralement l'adsorbant qu'il contient. Pendant ce remplacement, l'unité fonctionne ainsi en mode dégradé dans la mesure où le volume total d'adsorbant est réduit, ce qui peut se traduire par une élimination moins bonne des polluants. En outre, il s'avère que, souvent, l'adsorbant qui est prélevé aurait pu encore servir pour l'élimination des polluants car le suivi via la DCO n'est pas assez précis. Les stratégies existantes conduisent ainsi à une surconsommation de réactif et peuvent se révéler insatisfaisantes en termes de qualité de l'eau traitée.

Ces problématiques ne se posent pas dans les unités de traitement par oxydation dans lesquelles le réactif est ajouté en continu en une quantité suffisante pour l'élimination de polluants, et les techniques de traitement mises en œuvre sont beaucoup plus simples que les techniques d'adsorption.

## BREVE DESCRIPTION DE L'INVENTION

L'invention a pour objet une méthode de contrôle de l'élimination des micropolluants dans les eaux usées d'une unité de traitement dans laquelle les micropolluants sont éliminés par mise en contact des eaux usées avec au moins un réactif dans au moins un réacteur. La méthode selon l'invention est définie dans la revendication 1 et comprend entre autres les étapes suivantes :

(a) déterminer, entre une entrée de l'un des réacteurs de l'unité de traitement et une sortie du même réacteur ou d'un réacteur situé en aval de l'unité de traitement, au moins un indicateur $F_X$ de fluorescence représentatif de l'abattement d'un ou plusieurs des micropolluants à éliminer en fonction de l'unité de traitement, chaque indicateur $F_X$ correspondant à une intensité de fluorescence émise pour un couple ($\lambda x\_ex$ ; $\lambda x\_em$) d'une longueur d'onde d'excitation $\lambda x\_ex$ et d'une longueur d'onde d'émission $\lambda x\_em$,

(b) mesurer par spectroscopie de fluorescence 3D des eaux usées entrant par ladite entrée une première intensité de fluorescence $F_X\_in$ émise pour l'au moins un indicateur $F_X$,

(c) mesurer par spectroscopie de fluorescence 3D des eaux usées sortant par ladite sortie une deuxième intensité de fluorescence $F_X\_out$ émise pour l'au moins un indicateur $F_X$,

(d) calculer un pourcentage de diminution de l'intensité de fluorescence $\Delta F_X$ entre ladite entrée et ladite sortie pour chaque indicateur $F_X$ à partir des première et deuxième intensités de fluorescence préalablement mesurées $\Delta F_X = ((F_X\_in- F_X\_out)/F_X\_in)x100$,

(e) déterminer un pourcentage d'abattement Ab% entre ladite entrée et ladite sortie de l'au moins un micropolluant ou d'un ensemble des micropolluants à éliminer présents dans les eaux usées à partir d'une corrélation Ab%=f($\Delta F_X$) préalablement déterminée pour lesdites eaux usées exprimant le pourcentage d'abattement de l'au moins un micropolluant ou de l'ensemble des micropolluants à éliminer en fonction du pourcentage de diminution de l'intensité de fluorescence calculée d'un ou plusieurs indicateurs caractéristiques $F_X$,

(f) réguler la quantité de l'au moins un réactif injectée dans l'unité de traitement entre ladite entrée et ladite sortie en fonction du pourcentage d'abattement Ab% déterminé à l'étape (e).

L'invention est particulièrement adaptée pour le contrôle et/ou le suivi de l'abattement d'un ou plusieurs micropolluants par des unités de traitement par adsorption, utilisant typiquement comme réactif le charbon actif ou d'autres adsorbants (zéolithes, polymères, argiles, silices, oxydes métalliques, etc...), mais est également adaptée pour le contrôle et/ou le suivi de l'abattement d'un ou plusieurs micropolluants par des unités de traitement mettant en œuvre d'autres réactifs, telles que les unités de traitement par oxydation, utilisant typiquement l'ozone ou un précurseur de l'ozone comme réactif ou les unités de traitement par chloration, ou encore les unités d'oxydation avancée (procédés Fenton, photo-Fenton, UV + ozone, UV + $H_2O_2$, Ozone + $H_2O_2$, ...).

Dans un mode de réalisation, l'invention prévoit notamment d'injecter l'au moins un réactif de manière discontinue dans l'unité de traitement, l'étape (f) de régulation comprenant alors (i) aucune injection de l'au moins un réactif lorsque le pourcentage d'abattement déterminé pour un micropolluant ou pour un ensemble de micropolluants est supérieur à une valeur seuil prédéfinie ou (ii) une injection d'une quantité prédéfinie de l'au moins un réactif lorsque le pourcentage d'abattement déterminé pour un micropolluant ou pour un ensemble de micropolluants est inférieur à une valeur seuil prédéfinie et l'extraction d'une même quantité de l'au moins un réactif usé hors de l'unité de traitement.

Il est ainsi possible de maintenir à l'intérieur de l'unité une masse constante de l'au moins un réactif, ce qui est particulièrement avantageux pour les unités de traitement par adsorption dont l'au moins un réactif est un adsorbant. Le remplacement d'une partie du réactif en cours de fonctionnement de l'unité de traitement n'a ainsi aucune incidence sur la qualité de l'eau sortant de l'unité de traitement. Par ailleurs, les étapes (a) à (e) du procédé selon l'invention permettent un suivi précis de l'abattement d'un ou plusieurs micropolluants de sorte que l'ajout de réactif peut être effectué lorsque la masse de réactif nécessaire à l'élimination des micropolluants n'est plus suffisante. On comprend ainsi qu'il est possible d'ajuster précisément la quantité de réactif à ajouter afin de n'ajouter que la quantité de réactif nécessaire pour rétablir une masse suffisante à l'élimination des micropolluants. Cela permet d'une part de rallonger la durée d'utilisation de la masse de réactif présente dans l'unité de traitement, et d'autre part, de limiter la consommation de réactif.

Ainsi, dans un mode de réalisation particulièrement avantageux, la méthode de contrôle selon l'invention est une méthode de contrôle de l'élimination des micropolluants des eaux usées dans une unité de traitement par adsorption, et l'au moins un réactif est un adsorbant. De préférence, l'au moins un réactif est alors ajouté de manière discontinue dans au moins un des réacteurs de l'unité, tel que précédemment décrit. Avantageusement, l'au moins un réactif peut être du charbon actif en grains ou en poudre.

L'invention a également pour objet un système de contrôle de l'élimination d'au moins un micropolluant des eaux usées d'une unité de traitement comprenant au moins un réacteur, chaque réacteur présentant une entrée des eaux usées à traiter et une sortie des eaux usées traitées, le système étant défini dans la revendication 10 et comprenant entre autres :

- au moins une sonde de mesure par spectroscopie de fluorescence 3D, raccordée fluidiquement (i) à l'entrée de l'un des réacteurs de l'unité de traitement, (ii) à la sortie du même réacteur ou d'un réacteur situé en aval de l'unité de traitement, ou (iii) à la fois à ladite entrée et à ladite sortie via un organe de distribution, ladite au moins une sonde de mesure étant apte à déterminer une intensité de fluorescence pour au moins un indicateur,
- un système d'injection d'au moins un réactif dans l'unité de traitement,
- un système de gestion relié à l'au moins une sonde de mesure et au système d'injection. Le système de contrôle est entre autres configuré pour :

(i) déterminer entre ladite entrée et ladite sortie au moins un indicateur $F_X$ de fluorescence représentatif de l'abattement d'un ou plusieurs des micropolluants à éliminer en fonction de l'unité de traitement, chaque indicateur $F_X$ correspondant à une intensité de fluorescence émise pour un couple ($\lambda x\_ex$ ; $\lambda x\_em$) d'une longueur d'onde d'excitation $\lambda x\_ex$ et d'une longueur d'onde d'émission $\lambda x\_em$,

(ii) calculer un pourcentage de diminution de l'intensité de fluorescence $\Delta F_X$ entre ladite entrée et ladite sortie pour chaque indicateur $F_X$ à partir des première et deuxième intensités de fluorescence préalablement mesurées $\Delta F_X = ((F_{X\_in} - F_{X\_out})/F_{X\_in}).100$,

(iii) déterminer entre ladite entrée et ladite sortie un pourcentage d'abattement (Ab%) de l'au moins un micropolluant ou d'un ensemble des micropolluants à éliminer présents dans les eaux usées à partir d'une corrélation Ab%=f($\Delta F_X$) préalablement déterminée pour lesdites eaux usées exprimant le pourcentage d'abattement de l'au moins un micropolluant ou de l'ensemble des micropolluants à éliminer en fonction du pourcentage de diminution de l'intensité de fluorescence calculée d'un ou plusieurs indicateurs caractéristiques $F_X$,

(iv) réguler la quantité de l'au moins un réactif injectée par le système d'injection dans l'unité de traitement entre ladite entrée et ladite sortie en fonction du pourcentage d'abattement déterminé Ab%.

Avantageusement, le système selon l'invention est un système de contrôle de l'élimination d'au moins un micropolluant des eaux usées d'une unité de traitement par adsorption. De préférence, le système de gestion est alors configuré pour ajouter l'au moins un réactif de manière discontinue dans au moins un des réacteurs de l'unité, tel que précédemment décrit en référence à la méthode.

## DESCRIPTION DES FIGURES

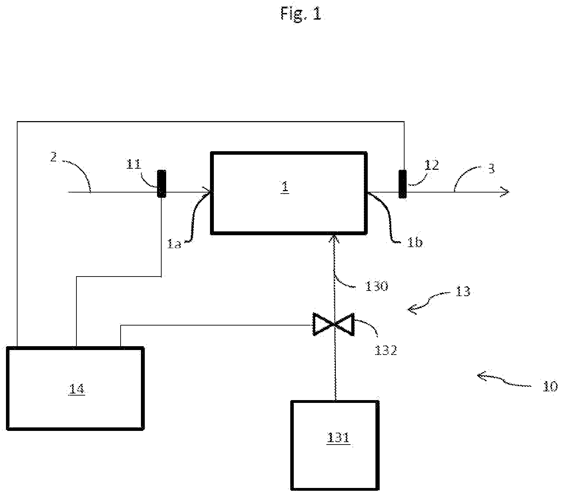

L'invention est maintenant décrite en référence aux dessins annexés, non limitatifs, dans lesquels la figure 1 représente schématiquement un système de contrôle selon un mode de réalisation de l'invention.

## DEFINITIONS

Le terme «micropolluant(s)» tel qu'utilisé dans la présente description désigne des substances organiques telles que les hormones, pesticides, herbicides, phtalates, résidus des produits pharmaceutiques, cosmétiques, de nettoyage, des métaux et leurs combinaisons. En particulier, les produits pharmaceutiques peuvent regrouper entre autres les familles de composés organiques de type analgésiques, antalgiques, antiépileptiques, antidépressifs, hormones, antibiotiques...

Ces micropolluants peuvent notamment comprendre les substances dont les législations nationales imposent l'élimination, telles que les substances mentionnées dans l'Ordonnance sur la protection des eaux (OEaux), (modification du 4 novembre 2015) par le Conseil Fédéral Suisse.

Ces micropolluants peuvent ainsi comprendre l'atenolol, l'amisulpride, l'amlodipine, l'amisulpride, l'azithromycine, le benzotriazole, le bicalutamide, la buprénorphine, le candésartan, la capécitabine, la carbamazépine, la ceftazidime, la ciprofloxacine, le citalopram, la clarithromycine, la cyprotérone, le déférasirox, le diclofénac, le disulfirame, le dronédarone, la duloxétine, l'éfavirenz, l'érythromycine, la fluoxétine, le fulvestrant, le furosémide, l'hydrochlorothiazide, l'ibuprofène, l'imidaclopride, l'irbésartan, le kétoprofène, le lanthanum, le méthylbenzotriazole (notamment le 4-méthylbenzotriazole et le 5 méthyl benzotriazole), le métoprolol, le mycophenolsyre, le naproxène, le nilotinib, l'ofloxacine, l'olanzapine, le paracétamol, le prednisolone, le propanolol, le propofol, la quétiapine, le sulfaméthoxazole, le tramadol, la venlafaxine, le cadmium, le chrome, le cuivre, le nickel, le mercure, le plomb, le zinc et leurs combinaisons.

En particulier, ces micropolluants peuvent comprendre l'amisulpride, l'amisulpride, l'azithromycine, le benzotriazole, le candésartan, la capécitabine, la carbamazépine, la ciprofloxacine, le citalopram, la clarithromycine, le diclofénac, l'érythromycine, l'hydrochlorothiazide, l'irbésartan, l'ibuprofène, l'imidaclopride, le kétoprofène, le méthylbenzotriazole (notamment le 4-méthylbenzotriazole et le 5 méthyl benzotriazole), le métoprolol, le naproxène, l'ofloxacine, le paracétamol, le propanolol, le sulfaméthoxazole, la venlafaxine, le cadmium, le chrome, le cuivre, le nickel, le mercure, le plomb, le zinc et leurs combinaisons.

L'expression « eaux usées » désigne des eaux polluées généralement par un usage humain. On peut distinguer les eaux usées domestiques (provenant des ménages), les eaux usées industrielles, les eaux usées agricoles et les eaux pluviales. Les eaux usées municipales ou urbaines peuvent être formées d'une ou plusieurs de ces eaux usées. Généralement, quand on parle d'eaux usées industrielles, on considère qu'il s'agit des eaux traitées dans une station d'épuration installée directement sur un site industriel et qui ne traite pas d'eaux usées domestiques ou pluviales.

## DESCRIPTION DETAILLEE DE L'INVENTION

Méthode de contrôle de l'élimination des micropolluants

Les inventeurs ont développé une méthode permettant de contrôler et/ou suivre, notamment en continu, l'abattement d'un ou plusieurs micropolluants dans les eaux usées d'une unité de traitement dans laquelle les micropolluants sont éliminés par mise en contact des eaux usées avec au moins un réactif dans au moins un réacteur.

La méthode selon l'invention comprend les étapes suivantes :

(a) déterminer, entre une entrée de l'un des réacteurs de l'unité de traitement et une sortie du même réacteur ou d'un réacteur situé en aval de l'unité de traitement, au moins un indicateur ($F_X$) de fluorescence représentatif de l'abattement d'un ou plusieurs des micropolluants à éliminer en fonction de l'unité de traitement, chaque indicateur ($F_X$) correspondant à une intensité de fluorescence émise pour un couple ($\lambda x\_ex$ ; $\lambda x\_em$) d'une longueur d'onde d'excitation ($\lambda x\_ex$) et d'une longueur d'onde d'émission ($\lambda x\_em$),

(b) mesurer par spectroscopie de fluorescence 3D des eaux usées entrant par ladite entrée une première intensité de fluorescence ($F_X\_in$) émise pour l'au moins un indicateur ($F_X$),

(c) mesurer par spectroscopie de fluorescence 3D des eaux usées sortant par ladite sortie une deuxième intensité de fluorescence ($F_X\_out$) émise pour l'au moins un indicateur ($F_X$),

(d) calculer un pourcentage de diminution de l'intensité de fluorescence ($\Delta F_X$) entre ladite entrée et ladite sortie pour chaque indicateur ($F_X$) à partir des première et deuxième intensités de fluorescence préalablement mesurées ($\Delta F_X = ((F_X\_in - F_X\_out)/F_X\_in).100$),

(e) déterminer un pourcentage d'abattement (Ab%) entre ladite entrée et ladite sortie de l'au moins un micropolluant ou d'un ensemble des micropolluants à éliminer présents dans les eaux usées à partir d'une corrélation Ab%=f($\Delta F_X$) préalablement déterminée pour lesdites eaux usées exprimant le pourcentage d'abattement de l'au moins un micropolluant ou de l'ensemble des micropolluants à éliminer en fonction du pourcentage de diminution de l'intensité de fluorescence calculée d'un ou plusieurs indicateurs caractéristiques ($F_X$),

(f) réguler la quantité de l'au moins un réactif injectée dans l'unité de traitement entre ladite entrée et ladite sortie en fonction du pourcentage d'abattement (Ab%) déterminé à l'étape (e).

Typiquement, les longueurs d'onde d'excitation ($\lambda x\_ex$) et les longueurs d'onde d'émission ($\lambda x\_em$) sont exprimées en nanomètres.

L'unité de traitement peut comprendre un, deux ou plusieurs réacteurs. Lorsqu'au moins deux réacteurs sont présents, ils peuvent être montés en série et/ou en parallèle. Les étapes (a) à (e) peuvent alors être mises en œuvre :

(i) à l'entrée et à la sortie d'au moins un réacteur de l'unité de traitement, notamment de chaque réacteur, ou

(ii) à l'entrée du réacteur le plus en amont et à la sortie du réacteur le plus en aval, ou,

(iii) à la fois à l'entrée et à la sortie d'au moins un réacteur de l'unité de traitement, notamment de chaque réacteur, et à l'entrée du réacteur le plus en amont et à la sortie du réacteur le plus en aval.

Dès lors, au cours de l'étape (f), on régule la quantité de l'au moins un réactif injectée dans l'unité de traitement entre ladite entrée et ladite sortie en fonction de chacun des pourcentages d'abattement (Ab%) déterminé, à savoir :

- dans le cas (i) en fonction du pourcentage d'abattement déterminé pour un ou plusieurs réacteurs de l'unité de traitement, de préférence en fonction du pourcentage d'abattement déterminé pour chacun des réacteurs de l'unité de traitement,
- dans le cas (ii), en fonction du pourcentage d'abattement déterminé entre l'entrée et la sortie de l'unité de traitement uniquement,
- dans le cas (iii), en fonction des différents pourcentages mentionnés pour les cas (i) et (ii).

Dans le cas (iii), on pourra notamment prévoir de réguler en priorité la quantité de réactif(s) ajoutée à l'un des réacteurs afin d'obtenir l'abattement souhaité en un ou plusieurs micropolluants en sortie de l'unité de traitement. Ce cas (iii) permet également de gérer les quantités de réactif(s) ajoutées à plusieurs réacteurs de manière étagée, en injectant du ou des réactifs dans un réacteur à la fois. On peut ainsi maintenir constant le pourcentage d'abattement de l'unité de traitement malgré des pourcentages d'abattement fluctuants dans chacun des réacteurs de l'unité de traitement. Ceci peut permettre d'optimiser l'utilisation du ou des réactifs.

Les étapes (a) à (f) sont avantageusement répétées de manière à suivre l'abattement d'un ou plusieurs micropolluants dans les eaux usées. Lorsque la qualité des eaux usées est constante, on pourra éventuellement se contenter de répéter uniquement les étapes (b) à (f) en utilisant les indicateurs ($F_X$) déterminés lors de l'étape (a), par exemple à la mise en route de l'unité de traitement. Il est toutefois préférable de réitérer l'ensemble des étapes.

L'ensemble des étapes (a) à (f) ou (b) à (f) est avantageusement répété dans le temps afin de suivre l'abattement des micropolluants dans les eaux usées, par exemple toutes les heures et /ou à un intervalle de temps égal ou supérieur à une période de temps correspondant au temps de séjour des eaux usées dans l'unité de traitement.

Un indicateur de fluorescence ($F_X$) correspond à une intensité de fluorescence émise pour un couple ($\lambda x\_ex$ ; $\lambda x\_em$) d'une longueur d'onde d'excitation ($\lambda x\_ex$) et d'une longueur d'onde d'émission ($\lambda x\_em$). Chaque indicateur ($F_X$) correspond ainsi à une fraction de matière organique dissoute pour laquelle une fluorescence peut être mesurée pour un couple ($\lambda x\_ex$ ; $\lambda x\_em$) donné. Typiquement, un indicateur de fluorescence correspond à un couple de longueurs d'onde ($\lambda x\_ex$ ; $\lambda x\_em$) pour lequel l'intensité de fluorescence émise est élevée, notamment plus élevée que pour d'autres couples de longueurs d'onde du spectre de fluorescence mesuré. De tels indicateurs peuvent être identifiés par analyse des spectres de fluorescence au moyen d'algorithmes bien connus de l'homme du métier, par exemple par la méthode d'analyse de données PARAFAC (Parallel Factor Analysis). On pourra ainsi utiliser dans la présente invention des indicateurs correspondant à des intensités de fluorescence émises pour des couples de longueurs d'onde ($\lambda x\_ex$ ; $\lambda x\_em$) déjà identifiés dans l'art antérieur, ou identifier de nouveaux indicateurs par analyse des spectres de fluorescence 3D.

L'invention peut ainsi comprendre une étape préalable d'identification d'indicateurs de fluorescence dans laquelle on mesure par spectroscopie de fluorescence 3D des intensités de fluorescence entre ladite entrée et ladite sortie pour une pluralité d'échantillons d'eaux usées et pour une pluralité de couples de longueurs d'onde ($\lambda x\_ex$ ; $\lambda x\_em$), on identifie le ou les couples de longueur ($\lambda x\_ex$ ; $\lambda x\_em$) pour lesquels l'intensité de fluorescence mesurée est la plus élevée, par exemple par la méthode PARAFAC, et on qualifie d'indicateurs les intensités émises pour le ou les couples de longueurs d'onde identifiés. Typiquement, ces indicateurs comprennent le couple pour lequel l'intensité de fluorescence est maximale ainsi que les couples dont l'intensité représente de 70 à 100% de cette intensité de fluorescence maximale.

Dans la présente invention, un indicateur ($F_X$) représentatif de l'abattement d'un ou plusieurs des micropolluants à éliminer correspond à une intensité de fluorescence émise pour un couple de longueurs d'onde ($\lambda x\_ex$ ; $\lambda x\_em$) susceptible d'être liée à la teneur en un ou plusieurs micropolluants à éliminer des eaux usées, de sorte qu'une corrélation peut être établie entre le pourcentage d'abattement de ce ou ces micropolluants et cet indicateur.

Un indicateur ($F_X$) représentatif de l'abattement d'un ou plusieurs des micropolluants peut être identifié (déterminé) au moyen d'une analyse statistique de données d'abattement d'un ou plusieurs micropolluants mesurées pour une unité de traitement entre ladite entrée et ladite sortie, ces données étant couplées à des données obtenues par une analyse spectroscopique de fluorescence 3D d'eaux usées contenant les micropolluants avant et après traitement (entre ladite entrée et ladite sortie), autrement dit à partir d'une analyse statistique de Matrices Emission-Excitation de Fluorescence, aussi désignées par l'acronyme MEEF (EEM en anglais).

La méthode selon l'invention comprend une étape préalable de construction d'une base de données contenant :

- des valeurs de pourcentages d'abattement du ou des micropolluants à éliminer calculées à partir de concentrations du ou des micropolluants mesurées, pour une pluralité d'échantillons d'eaux usées traitées dans l'unité de traitement entre ladite entrée et ladite sortie, et
- des valeurs de pourcentages de diminution des intensités de fluorescence mesurées, pour les mêmes échantillons d'eaux usées traitées dans la même unité de traitement entre la même entrée et la même sortie, par spectroscopie de fluorescence 3D pour une pluralité de couples de longueurs d'onde ($\lambda x\_ex$ ; $\lambda x\_em$).

Cette base de données peut comprendre des valeurs de pourcentages d'abattement et de diminution des intensités de fluorescence mesurées en entrée et sortie d'un ou plusieurs des réacteurs de l'unité de traitement, de préférence de chacun des réacteurs et/ou en entrée et en sortie de l'unité de traitement.

Typiquement, les valeurs contenues dans la base de données peuvent être des valeurs mesurées pour chaque micropolluant dont on souhaite contrôler l'abattement pour une unité de traitement ou pour différentes unités de traitement et pour une pluralité d'échantillons d'eaux usées à traiter. Pour chaque unité de traitement, on pourra mesurer ces valeurs pour une pluralité de quantités de réactif(s) utilisées et/ou pour une pluralité d'eaux usées. Ces valeurs peuvent être mesurées dans des unités de traitement industrielles existantes.

Autrement dit, la base de données peut aussi comprendre des données relatives à différentes unités de traitement (traitement par adsorption ou oxydation), aux quantités de réactif(s) ajoutées, à différentes qualités des eaux usées. Des eaux usées de qualité différente présentant par exemple une ou plusieurs des caractéristiques suivantes : des teneurs en micropolluants différentes, des teneurs en matières dissoutes différentes, des pH différents, des températures différentes, des compositions minérales différentes (présence d'ions susceptibles d'influer sur les conditions d'oxydo-réduction, l'alcalinité, la dureté, ...).

Typiquement, lors de la construction de la base de données, les concentrations en micropolluants peuvent être mesurées en utilisant des méthodes bien connues de l'homme du métier. Ces concentrations peuvent par exemple être mesurées par chromatographie en phase liquide ou gazeuse éventuellement couplée à une spectroscopie de masse.

L'abattement (Ab%) d'un micropolluant est défini par la formule Ab% =[(x-y)/x].100, Où x et y sont respectivement les concentrations du micropolluant avant et après traitement.

Les valeurs d'intensités de fluorescence peuvent être mesurées sur les échantillons d'eaux usées à traiter entrant et sortant par ladite entrée et ladite sortie de l'unité de traitement, en faisant varier la longueur d'onde d'excitation de 250 à 450nm et la longueur d'onde d'émission de 250 à 650nm.

On obtient ainsi pour chaque échantillon d'eaux usées à traiter deux matrices de fluorescence d'excitation/émission, l'une pour l'échantillon entrant par ladite entrée et l'autre pour le même échantillon sortant par ladite sortie. Chaque matrice va présenter un nombre de valeurs d'intensités de fluorescence qui va dépendre du pas entre deux longueurs d'onde successives d'excitation ou d'émission. On pourra par exemple faire varier la longueur d'onde d'excitation de 250 à 450nm avec un pas de 2nm et faire varier la longueur d'onde d'émission de 250 à 650nm avec un pas de 5nm. Bien entendu, d'autres valeurs de pas peuvent être envisagées.

A titre d'exemple, le tableau 1 donne les gammes de longueur d'onde d'excitation et d'émission pour les principaux composés fluorescents de la matière organique dissoute.

[Table 1]

| Type de composés | | Longueur d'onde d'excitation (nm) | Longueur d'onde d'émission (nm) |
|---|---|---|---|
| Protéines aromatiques | | 240-250 | 280-380 |
| Acides humiques | | 300-380 | 380-480 |
| Acides fulviques | | 240-280 | 380-480 |
| Produits microbiens solubles | Type tryptophane | 260-280 | 320-360 |
| | Type tyrosine | 260-280 | 300-320 |

On peut alors extraire de chaque matrice les intensités de fluorescence mesurées pour les différents couples ($\lambda x\_ex$ ; $\lambda x\_em$) de longueurs d'onde d'excitation/émission afin de calculer les pourcentages de diminution d'intensité contenus dans la base de données. L'homme du métier est à même de mettre en œuvre les mesures de fluorescence 3D afin d'obtenir les intensités des indicateurs de fluorescence des eaux usées entrant et sortant par ladite entrée et ladite sortie. Il pourra éventuellement prévoir de soustraire des valeurs de chaque matrice, les valeurs d'intensités de fluorescence obtenues de la même manière pour une eau distillée ou ultrapure, puis de normaliser les valeurs de la matrice en utilisant le pic Raman de cette eau distillée ou ultrapure afin d'obtenir des données de fluorescence en unité Raman. Il pourra également prévoir de corriger les intensités de fluorescence avec des facteurs de dilution lorsque nécessaire (Lawaetz, A.J., Stedmon, C.A., 2009. Fluorescence intensity calibration using the raman scatter peak of water. Appl. Spectrosc. 63, 936-940).

La base de données décrite précédemment peut être construite à partir d'intensités de fluorescences obtenues en balayant un ensemble de longueurs d'ondes d'excitation et d'émission tel que précédemment décrit. On peut également prévoir de construire une autre base de données contenant le même type de données mais uniquement pour une liste d'indicateurs de fluorescence préalablement déterminée et qui pourra être utilisée pour la détermination de la corrélation Ab%=f($\Delta F_X$) de l'étape (e). Cette autre base de données peut ainsi correspondre à une partie de la base de données précédemment décrite.

La méthode selon l'invention comprend en outre une étape préalable de détermination d'au moins une corrélation Ab%=f($\Delta F_X$), cette étape étant mise en œuvre après l'étape de construction d'une base de données, et comprenant :

(i) déterminer une pluralité de corrélations Ab%=f($\Delta F_X$) au moyen d'un modèle mathématique à partir de la base de données construite,
(ii) classer la pluralité de corrélations déterminées par ordre décroissant de précision de détermination du pourcentage d'abattement, la première corrélation correspondant à une corrélation optimale donnant une valeur d'abattement la plus proche des valeurs de pourcentages d'abattement mesurées pour l'au moins un micropolluant à éliminer et/ou pour un ensemble des micropolluants à éliminer.

Le modèle mathématique de l'étape (i) peut être choisi parmi un modèle de régression linéaire simple ou multiple, un modèle de régression polynomiale, des réseaux de neurones, du machine learning, .... De préférence, on pourra utiliser un même type de modèle pour déterminer la pluralité de corrélations.

Une corrélation peut corréler le pourcentage d'abattement d'un seul micropolluant à un pourcentage de diminution d'intensité d'un seul indicateur ou aux pourcentages de diminution d'intensité de plusieurs indicateurs. Une corrélation peut encore corréler le pourcentage d'abattement d'un ensemble ou de la totalité des micropolluants d'intérêt au pourcentage de diminution d'intensité d'un seul indicateur ou aux pourcentages de diminution d'intensité de plusieurs indicateurs.

Ainsi, une corrélation peut s'écrire :

$$Ab\% = f(\Delta F_i),$$

ou

$$Ab\% = f(\Delta F_i, \Delta F_j,),$$

ou encore

$$Ab\% = f(\Delta F_i, \Delta F_j, \Delta F_k, \ldots),$$

Où :

$\Delta F_i$, $\Delta F_j$, $\Delta F_k$ sont respectivement les variations de l'intensité de fluorescence des indicateurs $F_i$, $F_j$, $F_k$,
Ab% est le pourcentage d'abattement d'un micropolluant, d'un ensemble de micropolluants ou de la totalité des micropolluants, notamment des micropolluants d'intérêt. Dans ces deux derniers cas, le pourcentage d'abattement correspond alors à la moyenne des pourcentages d'abattement des micropolluants.

La pluralité de corrélations étant déterminée (établie), on pourra les classer (ii) par ordre décroissant de précision de détermination du pourcentage d'abattement pour un micropolluant, un ensemble de micropolluants ou la totalité des micropolluants.

Ceci peut être réalisé en effectuant une analyse statistique de la pluralité de corrélations, par exemple au moyen de coefficients de corrélation, ou autre. On pourra par exemple utiliser le coefficient de corrélation de Pearson pour les relations linéaires à un seul paramètre, et une analyse de régression partielle par les moindres carrés (Partial least squares regression) ou le critère d'information Bayésien pour les relations (linéaires ou non) à plusieurs paramètres.

Lors de l'étape (a), on peut alors déterminer l'au moins un indicateur ($F_X$) de fluorescence représentatif de l'abattement d'un ou plusieurs des micropolluants à éliminer à partir de la base de données préalablement construite. On pourra notamment déterminer cet au moins un indicateur ($F_X$) en fonction d'un ou plusieurs micropolluants que l'on souhaite éliminer.

La détermination de l'étape (a) peut ainsi être réalisée en fonction :

- d'un ou plusieurs micropolluants à éliminer, et
- de l'unité de traitement, et/ou
- de la qualité (des caractéristiques) des eaux usées à traiter.

En particulier, lors de l'étape (a), on peut déterminer comme indicateur ($F_X$) de fluorescence représentatif de l'abattement d'un ou plusieurs des micropolluants à éliminer, l'intensité de fluorescence émise pour le ou les couples de longueurs d'onde ($\lambda x\_ex$ ; $\lambda x\_em$) utilisés dans au moins la corrélation optimale, optionnellement dans les n premières corrélations, n étant un nombre entier non nul. Ce nombre n de corrélations retenu pourra être choisi par l'opérateur en fonction des micropolluants susceptibles d'être présents ponctuellement dans les eaux usées et dont l'intensité de fluorescence est proche d'un ou plusieurs indicateurs des premières corrélations. La ou les intensités de fluorescence peuvent être enregistrées dans une mémoire comme indicateur(s) de fluorescence représentatif de l'abattement d'un ou plusieurs des micropolluants à éliminer.

Avantageusement, lors de l'étape (e), la corrélation utilisée peut être la corrélation optimale, optionnellement une corrélation de rang inférieur.

La classification des corrélations peut notamment permettre de changer de corrélation quand un pourcentage d'abattement déterminé est aberrant, tel que décrit plus bas.

On notera que l'on pourra utiliser lors de l'étape (e) :

- une corrélation par micropolluant à éliminer, notamment une corrélation pour chacun des micropolluants à éliminer, ou
- une corrélation pour un ensemble de micropolluants à éliminer (par exemple pour la totalité des micropolluants).

Ceci peut permettre d'améliorer la régulation de la quantité de l'au moins un réactif.

En particulier, la corrélation utilisée à l'étape (e) peut être modifiée en cours de contrôle, notamment lorsqu'une valeur aberrante de pourcentage d'abattement est déterminée. La méthode selon l'invention peut alors comprendre l'étape additionnelle suivante (e') :

(i) déterminer une variation entre le pourcentage d'abattement déterminé et le pourcentage d'abattement en cours, notamment au moyen de la correction optimale, et comparer cette variation à une valeur seuil,
(ii) utiliser le pourcentage d'abattement ainsi déterminé à l'étape (f) si la variation est inférieure à la valeur seuil,
(iii) déterminer une autre valeur de pourcentage d'abattement si la variation est supérieure à la valeur seuil et utiliser cette autre valeur de pourcentage d'abattement à l'étape (f), l'autre valeur de pourcentage d'abattement étant déterminée à partir d'une autre corrélation Ab%=f($\Delta F_X$) préalablement déterminée.

Cette autre corrélation peut correspondre à une deuxième corrélation optimale donnant, après la corrélation optimale en cours, une valeur de pourcentage d'abattement la plus proche des valeurs de pourcentages d'abattement mesurées pour l'au moins un micropolluant à éliminer et/ou pour un ensemble des micropolluants à éliminer. Autrement dit, cette autre corrélation correspond à une corrélation de rang inférieur par rapport à la corrélation optimale.

Le pourcentage d'abattement en cours est défini comme le pourcentage d'abattement déterminé lors d'une itération précédente des étapes (a) à (f). De manière similaire, une corrélation optimale en cours est la corrélation optimale utilisée lors de l'itération précédente des étapes (a) à (f). Cette étape (e') peut être mise en œuvre après l'étape (e) et avant l'étape (f).

La valeur seuil permettant de déterminer si la variation est aberrante ou non peut être déterminée par l'opérateur par des essais, en faisant par exemple varier la qualité des eaux usées à traiter, notamment sa teneur en un composant dont on sait qu'il émet de la fluorescence pour des longueurs d'excitation et d'émission proches des indicateurs.

L'étape additionnelle (e') pourra être répétée avec les autres étapes. Autrement dit, la corrélation optimale peut être utilisée par défaut à l'étape (e), à chaque itération de l'ensemble des étapes, une corrélation de rang inférieur étant utilisée uniquement quand une valeur aberrante est déterminée.

Avantageusement, la méthode selon l'invention peut comprendre l'étape additionnelle suivante :

(i) mesurer à un instant t des concentrations en l'au moins un micropolluant dans les eaux à traiter entre ladite entrée et ladite sortie,
(ii) calculer les valeurs de pourcentages d'abattement entre ladite entrée et ladite sortie de l'au moins un micropolluant à partir des concentrations mesurées, et
(iii) calculer les pourcentages de diminution des intensités de fluorescences à partir des première et deuxième intensités de fluorescence mesurées au cours des étapes b) et c) au même instant t,
(iv) injecter dans la base de données les valeurs de pourcentages d'abattement et les pourcentages de diminution des intensités de fluorescence calculées à cet instant t.

Cette étape additionnelle permet d'enrichir la base de données lors de la mise en œuvre de la méthode de contrôle selon l'invention, permettant ainsi d'améliorer davantage la précision de la méthode au fil du temps en améliorant par exemple les corrélations.

Cette étape additionnelle peut être répétée à intervalles réguliers, par exemple 6 fois par an, des fréquences plus courtes ou plus longues étant cependant envisageables. On peut alors répéter au moins une des étapes de détermination de l'indicateur et de détermination d'au moins une corrélation, de préférence ces deux étapes, afin de mettre à jour la base de données et les corrélations.

Lorsque l'unité de traitement comprend deux réacteurs ou plus dont la quantité de réactif est régulée par le procédé selon l'invention, les étapes (i) et (ii) de l'étape préalable de détermination des corrélations sont alors mises en œuvre pour chacun des réacteurs dont la quantité de réactif(s) est régulée et /ou pour l'unité de traitement. Plusieurs indicateurs ($F_X$) de fluorescence représentatif de l'abattement d'un ou plusieurs des micropolluants à éliminer peuvent alors être déterminés pour chacun des réacteurs dont la quantité de réactif(s) est régulée et /ou pour l'unité de traitement. Pour chaque réacteur régulé et/ou pour l'unité de traitement, le choix des corrélations, la surveillance de valeurs de pourcentage d'abattement aberrantes et/ou l'enrichissement de la base de données, peuvent être effectués tel que précédemment décrit.

Les étapes (b) et (c) de mesure peuvent avantageusement être réalisées au moyen de d'une ou plusieurs sondes de mesure de fluorescence, notamment reliées à un système de gestion. Les autres étapes peuvent être réalisées au moyen d'un système de gestion. Lorsqu'une seule sonde est utilisée, la mesure à l'entrée et à la sortie est alors effectuée alternativement, par commande d'un organe de distribution de type vanne. Cette commande peut être réalisée par le système de gestion.

La régulation de la quantité de l'au moins un réactif injectée dans l'unité de traitement entre ladite entrée et ladite sortie en fonction du pourcentage d'abattement déterminé (étape f), peut être réalisée au moyen d'un système d'injection de réactif(s) servant à injecter l'au moins un réactif dans les eaux usées à traiter, par exemple au niveau de ladite entrée ou du réacteur situé immédiatement en aval de cette entrée, ce système d'injection étant relié au système de gestion.

Avantageusement, l'au moins un réactif étant injecté en continu dans l'unité de traitement, l'étape (f) de régulation de la méthode selon l'invention peut comprendre (i) une réduction de la quantité de l'au moins un réactif injectée lorsque le pourcentage d'abattement déterminé pour un micropolluant ou pour un ensemble de micropolluants est supérieur à une valeur seuil prédéfinie ou (ii) une augmentation de la quantité de l'au moins un réactif injectée lorsque le pourcentage d'abattement déterminé pour un micropolluant ou pour un ensemble de micropolluants est inférieur à une valeur seuil prédéfinie, ou (iii) un maintien de la quantité de l'au moins un réactif injectée lorsque le pourcentage d'abattement déterminé pour un micropolluant ou pour un ensemble de micropolluants est supérieur à une valeur seuil prédéfinie.

La réduction ou l'augmentation de l'au moins un réactif pourra être une réduction ou une augmentation d'une quantité constante prédéfinie, indépendante de la quantité en cours de réactif injectée. Cette quantité constante prédéfinie pourra être déterminée par l'opérateur de l'unité de traitement en fonction de son expérience du fonctionnement de l'unité de traitement, par exemple résultant d'observations des effets d'augmentations/réductions de la quantité de réactif.

Ainsi, par exemple, on pourra, à la mise en route de l'unité de traitement, injecter une quantité de l'au moins un réactif correspondant à une consigne initiale, puis, à compter de la deuxième itération des étapes (b) à (e), on peut déterminer si le pourcentage d'abattement déterminé pour un micropolluant ou pour un ensemble de micropolluants augmente, diminue ou est supérieur à la valeur seuil prédéfinie, et respectivement réduire, augmenter ou maintenir (i.e. ne pas modifier), la quantité de l'au moins un réactif injectée en cours.

Ce mode de réalisation pourra être mis en œuvre pour toute unité de traitement à l'intérieur de laquelle l'au moins un réactif est injecté en continu, et tout particulièrement pour une unité de traitement par oxydation à l'ozone et pour une unité de traitement par adsorption, notamment au charbon actif en poudre.

Avantageusement, l'au moins un réactif étant injecté de manière discontinue, l'étape (f) de régulation de la méthode selon l'invention peut comprendre (i) aucune injection de l'au moins un réactif lorsque le pourcentage d'abattement déterminé pour un micropolluant ou pour un ensemble de micropolluants est supérieur à une valeur seuil prédéfinie ou (ii) une injection d'une quantité prédéfinie de l'au moins un réactif lorsque le pourcentage d'abattement déterminé pour un micropolluant ou pour un ensemble de micropolluants est inférieur à une valeur seuil prédéfinie et l'extraction d'une même quantité de l'au moins un réactif usé hors de l'unité de traitement.

La quantité prédéfinie de l'au moins un réactif à ajouter pourra être déterminée par l'opérateur, notamment par des tests de l'unité de traitement ou par des tests en laboratoire ou sur des pilotes, ou au moyen de modèles préalablement établis. Elle peut correspondre à un pourcentage de la quantité totale de l'au moins un réactif présente dans l'unité de traitement. La quantité prédéfinie à ajouter peut notamment être inférieure à la quantité présente dans un réacteur de l'unité de traitement ou dans chaque réacteur de traitement. Il n'est ainsi pas nécessaire d'arrêter un réacteur pour renouveler partiellement ou totalement le réactif, comme c'est le cas aujourd'hui dans les unités de traitement par adsorption existantes.

Ce mode de réalisation est plus particulièrement adapté aux unités de traitement dans lesquelles une masse constante de l'au moins un réactif doit être maintenue à l'intérieur de l'unité de traitement et dans lesquelles l'au moins un réactif est ajouté de manière discontinue. C'est le cas typiquement des unités de traitement par adsorption fonctionnant avec du charbon actif en grains ou en poudre du type comprenant au moins un réacteur de traitement et dans lesquelles l'au moins un réactif est injecté dans au moins un des réacteurs.

L'extraction de l'au moins un réactif usé hors de l'unité de traitement peut être réalisée avant, pendant ou suite à l'injection de la quantité prédéfinie de l'au moins un réactif, de préférence après.

Dans les différents modes de réalisation, la valeur seuil prédéfinie peut correspondre à une valeur seuil imposée par une législation ou un utilisateur, en deçà de laquelle l'abattement est considéré comme insuffisant. Pour chacun de ces modes de réalisation, les valeurs seuils prédéfinies mentionnées peuvent être identiques ou non.

Avantageusement, on pourra prévoir d'enregistrer dans une mémoire, notamment intégrée au système de gestion, une ou plusieurs des données suivantes : la base de données, les indicateurs de fluorescence, notamment les indicateurs représentatifs de l'abattement d'un ou plusieurs des micropolluants à éliminer, y compris le cas échéant le ou les couples de longueurs d'onde associés, la ou les corrélations, notamment leur ordre de classement, les pourcentages de diminution des intensités de fluorescence, les pourcentages d'abattement déterminés, les différentes valeurs seuil.

La méthode de contrôle et/ou suivi de l'abattement des micropolluants organiques dans les eaux usées de l'invention est particulièrement adaptée au contrôle et/ou suivi de l'abattement des micropolluants dans les eaux usées urbaines, d'origine domestique ou industrielle, typiquement d'origine domestique et pouvant contenir des eaux industrielles, provenant, par exemple, des industries chimiques et pharmaceutiques.

Les eaux usées dont la méthode selon l'invention contrôle l'élimination de micropolluants peuvent être des eaux usées préalablement dépolluées, ou non, par traitement biologique.

Ce traitement biologique permet avantageusement de réduire la charge en matière organique (COD - carbone organique dissout). Le traitement biologique fait appel à une grande variété de microorganismes, dont des bactéries, qui

transform les matières biodégradables en produits simples tels que gaz carbonique, nitrate, azote et/ou biomasse.

La méthode de contrôle selon l'invention apporte de nombreux avantages. La mesure de l'intensité de fluorescence de plusieurs indicateurs $F_X$ alternativement ou simultanément par une ou plusieurs sondes de fluorescence en amont et en aval d'une unité de traitement permet de garantir le suivi et l'estimation de l'élimination des micropolluants sélectionnés ou l'élimination de micropolluants seuls en présence de substances organiques susceptibles d'interférer avec la mesure de l'intensité de fluorescence d'un indicateur $F_X$.

Plusieurs modèles de régression linéaire simples sont disponibles pour estimer les valeurs de pourcentage d'abattement des micropolluants à partir de plusieurs indicateurs $F_X$. Cela permet, lorsque le modèle ayant la meilleure estimation d'une valeur (par exemple coefficient $R^2$ le plus élevé) d'un indicateur $F_X$ n'est pas en mesure de donner des estimations correctes, de les garantir en utilisant un autre modèle d'un autre indicateur $F_X$.

Les indicateurs de fluorescence $F_X$ permettent de caractériser différentes fractions de matière organique dissoute telles que les biopolymères, les acides humiques, les acides fulviques ou les protéines aromatiques. Le suivi de l'intensité de fluorescence des indicateurs $F_X$ renseigne ainsi également sur l'évolution de la présence de ces fractions de matière organique dissoute dans les eaux usées avant et après traitement, apportant ainsi une information supplémentaire sur l'impact du traitement des micropolluants sur la qualité de l'eau traitée.

Système de contrôle de l'élimination des micropolluants

La méthode de contrôle de l'invention peut être mise en œuvre au moyen d'un système de contrôle selon l'invention, tel que défini dans la revendication 10.

Tel que représenté figure 1, ce système de contrôle 10 est destiné au contrôle de l'élimination des micropolluants d'une unité de traitement des eaux 1 laquelle comprend une entrée 1a pour l'entrée des eaux à traiter, et une sortie 1b pour l'évacuation des eaux traitées. Cette entrée 1a est par exemple en communication de fluide avec une conduite d'alimentation 2 des eaux usées à traiter et la sortie 1b en communication de fluide avec conduite d'évacuation 3 des eaux usées traitées.

Dans le mode de réalisation représenté figure 1, la méthode de contrôle est mise en œuvre entre l'entrée et la sortie de l'unité de traitement, cette dernière pouvant comprendre un ou plusieurs réacteurs montés en série et/ou en parallèle. La méthode de contrôle pourrait néanmoins être mise en œuvre pour un, plusieurs ou chacun des réacteurs de l'unité de traitement.

Le système de contrôle 10 représenté figure 1 comprend :

- une première sonde de mesure 11 située à l'entrée 1a de l'unité de traitement,
- une deuxième sonde de mesure 12 située à la sortie 1b de l'unité de traitement,
- un système d'injection 13 de réactif dans l'unité de traitement,
- un système de gestion 14.

Ces sondes de fluorescence 11, 12 sont adaptées à la mise en œuvre des étapes (b) et (c) de la méthode de contrôle selon l'invention. Il s'agit de sondes de mesure par spectroscopie de fluorescence 3D. L'homme du métier saura choisir des sondes appropriées capables d'effectuer des mesures aux longueurs d'ondes des indicateurs $F_X$.

Chacune des sondes 11, 12 est de préférence plongée dans les eaux usées à mesurer, avant et après l'unité de traitement, soit directement dans les conduites d'alimentation 2 et d'évacuation 3 des eaux usées de l'unité de traitement, tel que représenté figure 1, soit dans un canal de dérivation de ces conduites.

Les sondes mesurant l'intensité de fluorescence avant et après le traitement peuvent être reliées à une interface permettant l'affichage des intensités mesurées.

L'invention n'est pas limitée par un nombre de sondes de mesure. On notera qu'il est possible d'utiliser une unique sonde de mesure par spectroscopie de fluorescence 3D raccordée fluidiquement à l'entrée 1a et à la sortie 1b de l'unité de traitement par un organe de distribution de fluide de type vanne. La mesure des intensités à l'entrée et à la sortie est alors réalisée alternativement. En outre, une ou plusieurs sondes peuvent être utilisées pour contrôler plusieurs réacteurs de l'unité de traitement et/ou l'unité de traitement dans son ensemble (mesure entre entrée et sortie de l'unité de traitement)

Le système d'injection 13 de réactif sert à injecter le ou les réactifs dans les eaux usées à traiter, par exemple au niveau d'un réacteur de l'unité de traitement. Ce système d'injection 13 peut comprendre une ou plusieurs conduites d'alimentation 130 de l'unité de traitement en réactif(s), notamment à un ou plusieurs réacteurs de l'unité de traitement. Ces conduites 130 peuvent être reliées à une ou plusieurs capacités 131 contenant le réactif ou les réactifs. Le système d'injection 13 comprend en outre usuellement un moyen de réglage 132, par exemple une vanne de régulation, de la quantité de réactif(s) introduite à l'intérieur de l'unité de traitement.

Quel que soit le mode de réalisation de l'unité de traitement, le système de gestion 14 est configuré, notamment programmé, pour la mise en œuvre des étapes (a) et (d) à (f) de la méthode selon l'invention, ainsi que des étapes additionnelles de détermination des corrélations et d'enrichissement de la base de données. Il s'agit par exemple d'un système automatisé d'intégration et de conversion de données. Ce système de gestion peut être le système de gestion de l'unité de traitement ou de l'ensemble d'une installation de traitement des eaux usées comprenant l'unité de traitement. Il peut également être configuré pour commander la ou les sondes de mesure.

Le système de gestion 14 comprend typiquement un ou plusieurs processeurs, par exemple un microprocesseur, un microcontrôleur ou autre. Il comprend également des interfaces de sortie ou d'entrée/sortie. Il peut s'agit d'interfaces interfaces de communication sans fil (Bluetooth, WIFI ou autre) ou des connecteurs (port réseau, port USB, port série, port Firewire®, port SCSI ou autre). Ces interfaces d'entrée et/ou sortie peuvent former des moyens de communication, optionnellement bidirectionnels, entre le système de gestion, la ou les sondes de fluorescence et/ou le système d'injection.

Le système de gestion peut également comprendre des moyens de stockage qui peuvent être une mémoire vive (RAM), une mémoire morte programmable effaçable électriquement (EEPROM), une mémoire flash, une mémoire externe ou autre. Ces moyens de stockage peuvent, entre autres, stocker des données reçues, les valeurs mesurées, les valeurs calculées, la base de données, les corrélations, et un ou plusieurs programmes informatiques.

La ou les sondes 11, 12 mesurant l'intensité de fluorescence avant et après le traitement sont ainsi reliées au système de gestion 14, de même que le système d'injection 13. Les intensités de fluorescence mesurées par la ou les sondes sont transmises au système de gestion qui calcule une variation ($\Delta F_X$) de l'intensité de fluorescence pour chaque indicateur ($F_X$), correspondant, dans l'exemple de la figure 1, à la différence entre l'intensité de fluorescence avant traitement et l'intensité de fluorescence après traitement. Le système calcule ensuite, à partir de cette variation, le pourcentage d'abattement des micropolluants en appliquant la corrélation Ab%=f($\Delta F_X$) enregistrée dans le système. Le système régule enfin la quantité de réactif(s).

A cet effet, le système de gestion peut par exemple être configuré, notamment programmé, pour :

(i) comparer le pourcentage d'abattement déterminé avec le pourcentage d'abattement en cours et avec une valeur seuil prédéfinie,
(ii) déterminer une consigne de quantité de réactif(s) à injecter dans l'unité de traitement consistant en (i) une réduction de la quantité de réactif injectée lorsque le pourcentage d'abattement déterminé pour un micropolluant ou pour un ensemble de micropolluants est supérieur à la valeur seuil prédéfinie ou (ii) une augmentation de la quantité de réactif(s) injectée lorsque le pourcentage d'abattement déterminé pour un micropolluant ou pour un ensemble de micropolluants est inférieur à la valeur seuil prédéfinie, ou (iii) un maintien de la quantité de réactif(s) injectée lorsque le pourcentage d'abattement déterminé pour un micropolluant ou pour un ensemble de micropolluants est supérieur à la valeur seuil prédéfinie et
(iii) transmettre la consigne au système d'injection 13.

Alternativement, le système peut par exemple être configuré, notamment programmé, pour :

(i) comparer le pourcentage d'abattement déterminé avec une valeur seuil,
(ii) déterminer une consigne de quantité de réactif(s) à injecter dans l'unité de traitement consistant en (i) aucune injection de réactif(s) lorsque le pourcentage d'abattement déterminé pour un micropolluant ou pour un ensemble de micropolluants est supérieur à la valeur seuil prédéfinie ou (ii) une injection d'une quantité prédéfinie de réactif(s) lorsque le pourcentage d'abattement déterminé pour un micropolluant ou pour un ensemble de micropolluants est inférieur à la valeur seuil prédéfinie et une consigne d'extraction de la même quantité de réactif(s) hors de l'unité de traitement,
(iii) transmettre la consigne au système d'injection 13 et la consigne d'extraction à un système d'extraction de réactif de l'unité de traitement.

On pourra ainsi prévoir un système d'extraction relié au système de gestion pour chaque réacteur. Ce système d'extraction est, par exemple, une vanne sur une conduite d'extraction connectée à un réacteur de l'unité de traitement, ou tout autre dispositif permettant d'extraire un réactif, notamment un réactif de type adsorbant, d'un réacteur.

Les étapes décrites ci-dessus peuvent être mises en œuvre pour un ou plusieurs pourcentages d'abattement déterminés entre l'entrée et la sortie de l'unité de traitement et/ou entre l'entrée et la sortie d'un ou plusieurs des réacteurs de l'unité de traitement et/ou entre l'entrée et la sortie de chacun des réacteurs de l'unité de traitement.

Afin de permettre un suivi en continu des données et de consulter des historiques journaliers, mensuels ou annuels de l'unité de traitement, le système de gestion peut inclure ou être relié à une interface permettant d'afficher les intensités de fluorescence mesurées par la ou les sondes, la variation de ces intensités et/ou le pourcentage d'abattement des micropolluants.

Lorsque chacun des réacteurs de l'unité de traitement est suivi en entrée et en sortie par la méthode décrite, de même que l'unité de traitement entre son entrée et sa sortie, le système selon l'invention permet d'apporter les informations nécessaires à une gestion du renouvellement étagé d'un ensemble ou d'une batterie de réacteurs (N réacteurs devant atteindre X% d'élimination globale). Il est alors possible grâce à l'invention de maintenir ce X% d'élimination globale avec des abattements fluctuant parmi les N réacteurs. Les capacités du ou des réactifs sont alors utilisées de manière optimale. Ce renouvellement étagé est particulièrement avantageux pour les unités de traitement par adsorption. L'invention permet en effet le fonctionnement en continu d'une unité de traitement par adsorption en maintenant une masse constante de réactif à l'intérieur de l'unité malgré un renouvellement du réactif.

**Exemples**

**Exemple 1**

On sélectionne un groupe de substances dont on souhaite contrôler l'élimination, par exemple les 12 substances faisant partie des substances à contrôler dans les eaux usées urbaines par la législation suisse et figurant dans l'Ordonnance sur la protection des eaux (OEaux), (modification du 4 novembre 2015) du Conseil Fédéral Suisse.

Un nombre statistiquement représentatif de tests ont été menés dans une unité de traitement par adsorption comprenant quatre réacteurs de charbon activés en grains (CAG) montés en parallèle. Les réacteurs sont alimentés en eaux usées par une conduite d'alimentation commune et les eaux usées traitées sortant de chaque réacteur sont réunies dans une conduite d'évacuation commune. Deux sondes de fluorescence sont ici positionnées sur les conduites d'alimentation et d'évacuation respectivement.

L'analyse statistique de ces tests a permis de déterminer les indicateurs représentatifs de l'abattement des 12 substances suivants :

- un premier indicateur $F_1$ correspondant au couple de longueurs d'onde ($\lambda x\_ex = 325$ nm, $\lambda x\_em = 385$ nm), pour lequel la relation suivante a été déterminée au moyen d'un modèle de simple régression linéaire :

$$\%MP_1 = a_1 . \Delta F_1 + b_1, (C1)$$

Le coefficient de Pearson de cette régression linéaire est de $R^2 = 0.90$.

- un deuxième indicateur $F_2$ correspondant au couple de longueurs d'onde ($\lambda x\_ex = 250$ nm, $\lambda x\_em = 420$ nm), pour lequel la relation suivante a été déterminée au moyen d'un modèle de simple régression linéaire :

$$\%MP_2 = a_2 . \Delta F_2 + b_2, (C2)$$

Le coefficient de Pearson de cette régression linéaire est de $R^2 = 0,85$.

Dans les relations C1 et C2 décrites ci-dessus :

$\%MP_i$ : pourcentage d'abattement moyen des 12 substances, calculé à partir de l'indicateur $F_i$,
$\Delta F_i$ : pourcentage de diminution d'intensité de fluorescence de l'indicateur $F_i$ avant et après le traitement,
$a_i$ : pente de la régression linéaire établie pour l'indicateur $F_i$,
$b_i$ : intersection avec l'axe des ordonnées de la corrélation établie pour l'indicateur $F_i$.

Dans cet exemple, l'indicateur $F_i$ le plus représentatif de l'abattement des 12 substances (celui dont la corrélation donne une valeur d'abattement la plus proche des valeurs de pourcentages d'abattement mesurées pour les 12 substances) est donc l'indicateur $F_1$. Une relation linéaire similaire a été établie en remplaçant les mesures de fluorescence 3D par des mesures d'absorption UV (254nm) dans les mêmes conditions de traitement. Le coefficient de Pearson de la régression linéaire obtenue est $R^2 = 0,74$.
L'utilisation de la fluorescence 3D permet ainsi d'améliorer l'estimation de l'abattement pour ces 12 substances.

**Exemple 2**

On sélectionne un produit pharmaceutique dont on souhaite contrôler l'élimination, ici l'atenolol.
Un nombre statistiquement représentatif de tests ont été menés dans la même unité de traitement que celle de l'exemple 1.
L'analyse statistique de ces tests a permis de déterminer comme indicateur $F_1$ représentatif de l'abattement de l'atenolol le même couple de longueurs d'onde ($\lambda x\_ex = 325$ nm, $\lambda x\_em = 385$ nm), pour lequel la relation suivante a été déterminée au moyen d'un modèle de simple régression linéaire :

$$\%MP_1' = a_1'. \Delta F_1 + b_1', (C3)$$

Où :

$\%MP_1'$ : pourcentage d'abattement de l'atenolol, exprimé en pourcentage,
$\Delta F_1$ : pourcentage de diminution d'intensité de fluorescence de l'indicateur $F_1$ avant et après le traitement, également exprimée en pourcentage,
$a_1'$ : pente de la régression linéaire,
$b_1'$ : intersection avec l'axe des ordonnées.

Le coefficient de Pearson de cette régression linéaire est de $R^2 = 0.88$.
Une relation linéaire similaire a été établie en remplaçant les mesures de fluorescence 3D par des mesures d'absorption UV (254nm) dans les mêmes conditions de traitement. Le coefficient de Pearson obtenu est $R^2 = 0,59$.
L'utilisation de la fluorescence 3D permet également d'améliorer le contrôle de l'abattement ce produit.

## Exemple 3

Des eaux usées sont traitées dans l'unité de traitement de l'exemple 1.
Les eaux usées à traitées dans cet exemple peuvent contenir ponctuellement des quantités élevées de protéines susceptibles d'émettre de la fluorescence aux longueurs d'ondes d'émission et d'excitation proches de l'indicateur $F_1$. On mesure alors des intensités de fluorescence plus élevées que les valeurs usuelles, conduisant à un pourcentage d'abattement erroné.
Dans ce cas, lorsque le système détecte une valeur aberrante du pourcentage d'abattement, il détermine une nouvelle valeur du pourcentage d'abattement pour une corrélation autre que la corrélation optimale donnant une valeur d'abattement la plus proche des valeurs de pourcentages d'abattement mesurées pour les 12 substances, ici en appliquant la relation C2 $\%MP_2 = a_2. \Delta F_2 + b_2$ déterminée dans l'exemple 1.

## Exemple 4

Une analyse statistique des tests de l'exemple 1 a permis d'établir par un modèle de régression linéaire multiple la corrélation suivante :

$$\%MP = \Delta F_1.a_1 + \Delta F_2.a_2 + \Delta F_3.a_3 + b \ (C4)$$

Où :

$\%MP$ : pourcentage d'abattement moyen des 12 substances, exprimé en pourcentage,
$\Delta F_i$ : pourcentage de diminution d'intensité de fluorescence de l'indicateur $F_i$ avant et après le traitement, les indicateurs $F_1$ et $F_2$ étant ceux déterminés dans l'exemple 1, l'indicateur $F_3$ correspondant à l'intensité de fluorescence émise pour le couple de longueurs d'onde ($\lambda x\_ex = 340$ nm, $\lambda x\_em = 430$ nm),
$a_i$ : coefficient du paramètre $\Delta F_i$ ,
$b$ : intersection avec l'axe des ordonnées.

Le coefficient de Pearson de cette relation est $R^2 = 0,98$, bien supérieur aux coefficients de Pearson calculés pour les corrélations C1 et C2 fonctions uniquement des indicateurs $F_1$ ou $F_2$. Ainsi, le pourcentage d'abattement moyen des 12 substances peut être déterminé encore plus précisément à partir des trois indicateurs $F_1$, $F_2$, $F_3$.

## Exemple 5

Dans cet exemple, on contrôle les performances d'abattement des 12 micropolluants dont le suivi est imposé par la législation suisse dans l'unité de traitement de l'exemple 1. Chacun des réacteurs est équipé d'un injecteur de charbon actif en grains. Du CAG frais est injecté de manière discontinue dans l'unité. De manière usuelle, chaque réacteur reçoit du CAG frais à tour de rôle.
Les sondes de fluorescence positionnées en amont et en aval de l'unité de traitement mesurent en temps réel les intensités de fluorescence de l'indicateur $F_1$.
Le système calcule ensuite un pourcentage de diminution $\Delta F_1$ de l'intensité de fluorescence de l'indicateur $F_1$ déterminé dans l'exemple 1, puis le pourcentage d'abattement en utilisant la relation (C1) $\%MP_1 = a_1. \Delta F_1 + b_1$.

Si le pourcentage d'abattement ainsi déterminé est proche du seuil de 80% requis par la législation suisse, notamment s'il diminue et devient par exemple inférieur à 81%, le système injecte dans le premier réacteur de l'unité de traitement du CAG frais une quantité fixée par l'opérateur correspondant par exemple à 1% de la masse totale de CAG de ce premier réacteur (d'autres quantités sont toutefois envisageables et seront adaptées par l'opérateur en fonction du fonctionnement de l'unité de traitement). Après une durée correspondant au temps de séjour des eaux usées dans l'unité de traitement, par exemple 15 minutes, le système détermine un pourcentage d'abattement de 81% et injecte une nouvelle quantité de CAG frais dans le deuxième réacteur (par exemple représentant 1% de la masse totale de CAG du deuxième réacteur). Après une durée correspondant au temps de séjour dans l'unité de traitement, le système détermine à nouveau le pourcentage d'abattement. S'il est de 83%, le système n'injecte pas de CAG frais. Le système va alors déterminer régulièrement le pourcentage d'abattement et injectera du CAG frais lors d'une nouvelle diminution de ce pourcentage d'abattement à 82%, en raison de la saturation progressive du CAG.

Dans ce type d'unité de traitement, le système extrait de chaque réacteur dans lequel du CAG frais a été injecté, une quantité de CAG identique à la quantité ajoutée afin de maintenir une masse de réactif constante à l'intérieur de chaque réacteur. Cette extraction peut avantageusement être réalisée après un lavage du lit de CAG du réacteur, afin d'éviter d'extraire des matières en suspension/biomasse à la place du CAG, et en fluidisation.

**Exemple 6**

Dans cet exemple, on contrôle les performances d'abattement des 12 micropolluants dont le suivi est imposé par la législation suisse d'une unité de traitement par ozonation comprenant typiquement un réacteur équipé d'une conduite d'amenée des eaux usées, d'une conduite d'évacuation des eaux usées traitées et d'une conduite d'injection d'ozone. L'ozone est injecté en continu dans le réacteur. Les sondes de fluorescence positionnées en amont et en aval de l'unité de traitement (respectivement sur les conduites d'alimentation et d'évacuation) mesurent en temps réel les intensités de fluorescence de l'indicateur $F_1$.

L'unité de traitement fonctionne par exemple avec une injection d'ozone de 0,35 $gO_3$/gCOD (COD : carbone organique dissous). Le système détermine le pourcentage d'abattement de l'unité à intervalles de temps réguliers. Si le pourcentage d'abattement diminue à 81%, le système va augmenter quantité d'ozone injectée d'une certaine valeur la quantité d'ozone injectée afin d'éviter d'atteindre le seuil de 80%. Cette valeur est définie par l'opérateur de l'unité de traitement et peut par exemple être de 0,01 $gO_3$/gCOD (d'autres valeurs sont toutefois envisageables et seront adaptées par l'opérateur en fonction du fonctionnement de l'unité de traitement), de sorte que la quantité finale d'ozone est de 0,36 $gO_3$/gCOD. Après une durée correspondant au temps de séjour des eaux usées dans l'unité de traitement, par exemple 15min, le système détermine un pourcentage d'abattement de 82%. La quantité d'ozone injectée est correcte, le système ne modifie pas cette quantité jusqu'à ce qu'il détermine :

- un pourcentage d'abattement de 81%, dans ce cas, il va à nouveau augmenter la quantité d'ozone pour atteindre 0,37 $gO_3$/gCOD, ou
- un pourcentage d'abattement de 83%, dans ce cas le système réduit la quantité d'ozone pour atteindre 0,35 $gO_3$/gCOD afin d'éviter de faire fonctionner l'unité dans des conditions de sur-qualité qui ne sont pas souhaitables.

Le contrôle de l'élimination en micropolluants des eaux usées d'une unité de traitement présentant un réacteur dans lequel est injecté en continu du charbon actif en poudre (CAP) peut être réalisé de manière similaire.

**Revendications**

1. Méthode de contrôle de l'élimination des micropolluants des eaux usées dans une unité de traitement dans laquelle les micropolluants sont éliminés par mise en contact des eaux usées avec au moins un réactif dans au moins un réacteur, comprenant les étapes suivantes :

   (a) déterminer, entre une entrée de l'un des réacteurs de l'unité de traitement et une sortie du même réacteur ou d'un réacteur situé en aval de l'unité de traitement, au moins un indicateur $F_X$ de fluorescence représentatif de l'abattement d'un ou plusieurs des micropolluants à éliminer en fonction de l'unité de traitement, chaque indicateur $F_X$ correspondant à une intensité de fluorescence émise pour un couple d'une longueur d'onde d'excitation $\lambda x\_ex$ et d'une longueur d'onde d'émission $\lambda x\_em$,
   (b) mesurer par spectroscopie de fluorescence 3D des eaux usées entrant par ladite entrée une première intensité de fluorescence $F_{X\_in}$ émise pour l'au moins un indicateur $F_X$,
   (c) mesurer par spectroscopie de fluorescence 3D des eaux usées sortant par ladite sortie une deuxième intensité de fluorescence $F_{X\_out}$ émise pour l'au moins un indicateur $F_X$,

(d) calculer un pourcentage de diminution de l'intensité de fluorescence $\Delta F_X$ entre ladite entrée et ladite sortie pour chaque indicateur $F_X$ à partir des première et deuxième intensités de fluorescence préalablement mesurées : $\Delta F_X = ((F_{X\_in} - F_{X\_out})/F_{X\_in}).100$,

(e) déterminer un pourcentage d'abattement Ab% entre ladite entrée et ladite sortie de l'au moins un micropolluant ou d'un ensemble des micropolluants à éliminer présents dans les eaux usées à partir d'une corrélation Ab%=f($\Delta F_X$) préalablement déterminée pour lesdites eaux usées exprimant le pourcentage d'abattement de l'au moins un micropolluant ou de l'ensemble des micropolluants à éliminer en fonction du pourcentage de diminution de l'intensité de fluorescence calculée d'un ou plusieurs indicateurs caractéristiques $F_X$,

(f) réguler la quantité de l'au moins un réactif injectée dans l'unité de traitement entre ladite entrée et ladite sortie en fonction du pourcentage d'abattement Ab% déterminé à l'étape (e),

la méthode comprenant :

1) une étape préalable de construction d'une base de données contenant :

- des valeurs de pourcentages d'abattement du ou des micropolluants à éliminer calculées à partir de concentrations du ou des micropolluants mesurées pour une pluralité d'échantillons d'eaux usées traitées dans l'unité de traitement entre ladite entrée et ladite sortie, et
- des valeurs de pourcentages de diminution des intensités de fluorescence mesurées, pour les mêmes échantillons d'eaux usées traitées dans la même unité de traitement entre la même entrée et la même sortie, par spectroscopie de fluorescence 3D pour une pluralité de couples d'une longueur d'onde d'excitation $\lambda x\_ex$ et d'une longueur d'onde d'émission $\lambda x\_em$, et

2) une étape préalable de détermination d'au moins une corrélation Ab%=f($\Delta F_X$), cette étape étant mise en œuvre après ladite étape de construction d'une base de données, et comprenant :

(i) déterminer une pluralité de corrélations Ab%=f($\Delta F_X$) au moyen d'un modèle mathématique à partir de ladite base de données construite,
(ii) classer la pluralité de corrélations déterminées par ordre décroissant de précision de détermination du pourcentage d'abattement, la première corrélation correspondant à une corrélation optimale donnant une valeur d'abattement la plus proche des valeurs de pourcentages d'abattement mesurées pour l'au moins un micropolluant à éliminer et/ou pour un ensemble des micropolluants à éliminer.

2. Méthode de contrôle selon la revendication 1, dans laquelle l'unité de traitement comprend au moins deux réacteurs montés en série et/ou en parallèle et les étapes (a) à (e) sont mises en œuvre :

(i) à l'entrée et à la sortie d'au moins un réacteur de l'unité de traitement, ou
(ii) à l'entrée du réacteur le plus en amont et à la sortie du réacteur le plus en aval, ou,
(iii) à la fois à l'entrée et à la sortie d'au moins un réacteur de l'unité de traitement et à l'entrée du réacteur le plus en amont et à la sortie du réacteur le plus en aval, et au cours de l'étape (f), on régule la quantité de l'au moins un réactif injectée dans l'unité de traitement entre ladite entrée et ladite sortie en fonction de chacun des pourcentages d'abattement Ab% déterminé.

3. Méthode de contrôle selon la revendication 1 ou la revendication 2, dans laquelle, l'étape (a) comprend : déterminer comme indicateur $F_X$ de fluorescence représentatif de l'abattement d'un ou plusieurs des micropolluants à éliminer, l'intensité de fluorescence émise pour le ou les couples d'une longueur d'onde d'excitation $\lambda x\_ex$ et d'une longueur d'onde d'émission $\lambda x\_em$ utilisé(s) au moins dans la corrélation optimale, optionnellement dans les n premières corrélations, n étant un nombre entier non nul.

4. Méthode de contrôle selon l'une quelconque des revendications 1 à 3, dans laquelle au cours de l'étape (e), la corrélation Ab%=f($\Delta F_X$) utilisée pour déterminer le pourcentage d'abattement est la corrélation optimale, optionnellement une corrélation de rang inférieur.

5. Méthode de contrôle selon la revendication 4, comprenant l'étape additionnelle suivante (e') :

(i) déterminer une variation entre le pourcentage d'abattement déterminé et le pourcentage d'abattement en cours et comparer cette variation à une valeur seuil,
(ii) utiliser le pourcentage d'abattement ainsi déterminé à l'étape (f) si la variation est inférieure à la valeur seuil,

(iii) déterminer une autre valeur de pourcentage d'abattement si la variation est supérieure à la valeur seuil et utiliser cette autre valeur de pourcentage d'abattement à l'étape (f), l'autre valeur de pourcentage d'abattement étant déterminée à partir d'une autre corrélation Ab%=f($\Delta F_X$) préalablement déterminée.

6. Méthode de contrôle selon l'une quelconque des revendications 1 à 5, comprenant l'étape additionnelle suivante :

(i) mesurer à un instant t des concentrations en l'au moins un micropolluant dans les eaux à traiter entre ladite entrée et ladite sortie,
(ii) calculer les valeurs de pourcentages d'abattement entre ladite entrée et ladite sortie de l'au moins un micropolluant à partir des concentrations mesurées,
(iii) calculer les pourcentages de diminution des intensités de fluorescences à partir des première et deuxième intensités de fluorescence mesurées au cours des étapes (b) et (c) au même instant t, et
(iv) injecter dans la base de données les valeurs d'abattement et les pourcentages de diminution des intensités de fluorescence calculées à cet instant t.

7. Méthode de contrôle selon l'une quelconque des revendications 1 à 6, dans laquelle, l'au moins un réactif étant injecté en continu dans l'unité de traitement, l'étape (f) de régulation comprend (i) une réduction de la quantité de l'au moins un réactif injectée lorsque le pourcentage d'abattement déterminé pour un micropolluant ou pour un ensemble de micropolluants est supérieur à une valeur seuil prédéfinie, ou (ii) une augmentation de la quantité de l'au moins un réactif injectée lorsque le pourcentage d'abattement déterminé pour un micropolluant ou pour un ensemble de micropolluants est inférieur à une valeur seuil prédéfinie, ou (iii) un maintien de la quantité de l'au moins un réactif injectée lorsque le pourcentage d'abattement déterminé pour un micropolluant ou pour un ensemble de micropolluants est supérieur à une valeur seuil prédéfinie.

8. Méthode de contrôle selon l'une quelconque des revendications 1 à 7, dans laquelle, l'au moins un réactif étant injecté de manière discontinue dans l'unité de traitement, l'étape (f) de régulation comprend (i) aucune injection de l'au moins un réactif lorsque le pourcentage d'abattement déterminé pour un micropolluant ou pour un ensemble de micropolluants est supérieur à une valeur seuil prédéfinie ou (ii) une injection d'une quantité prédéfinie de l'au moins un réactif lorsque le pourcentage d'abattement déterminé pour un micropolluant ou pour un ensemble de micropolluants est inférieur à une valeur seuil prédéfinie et l'extraction d'une même quantité de l'au moins un réactif usé hors de l'unité de traitement.

9. Méthode de contrôle selon l'une quelconque des revendications 1 à 8, dans laquelle les étapes (b) et (c) sont réalisées au moyen d'au moins une sonde de mesure de fluorescence reliée à un système de gestion.

10. Système de contrôle (10) de l'élimination d'au moins un micropolluant des eaux usées d'une unité de traitement (1) comprenant au moins un réacteur, chaque réacteur présentant une entrée (1a) des eaux usées à traiter et une sortie (1b) des eaux usées traitées, le système comprenant:

- au moins une sonde de mesure (11, 12) par spectroscopie de fluorescence 3D, raccordée fluidiquement (i) à l'entrée de l'un des réacteurs de l'unité de traitement, (ii) à la sortie du même réacteur ou d'un réacteur situé en aval de l'unité de traitement, ou (iii) à la fois à ladite entrée et à ladite sortie via un organe de distribution, ladite au moins une sonde de mesure (11,12) étant configurée pour : (i) déterminer pour au moins un indicateur $F_X$ de fluorescence représentatif de l'abattement d'un ou plusieurs des micropolluants à éliminer en fonction de l'unité de traitement, chaque indicateur $F_X$ correspondant à une intensité de fluorescence émise pour un couple d'une longueur d'onde d'excitation $\lambda x\_ex$ et d'une longueur d'onde d'émission $\lambda x\_em$, une première intensité de fluorescence $F_X\_in$ émise par les eaux usées entrant par ladite entrée et une deuxième intensité de fluorescence $F_X\_out$ émise par les eaux usées sortant par ladite sortie,
- un système d'injection (13) d'au moins un réactif dans l'unité de traitement (1),
- un système de gestion (14) relié à ladite au moins une sonde de mesure (11,12) et au système d'injection (13), configuré pour :

(ii) calculer un pourcentage de diminution de l'intensité de fluorescence $\Delta F_X$ entre ladite entrée et ladite sortie pour chaque indicateur $F_X$ à partir des première et deuxième intensités de fluorescence préalablement mesurées $\Delta F_X = ((F_X\_in-F_X\_out)/F_X\_in).100$,
(iii) déterminer entre ladite entrée et ladite sortie un pourcentage d'abattement Ab% de l'au moins un micropolluant ou d'un ensemble des micropolluants à éliminer présents dans les eaux usées à partir d'une corrélation Ab%=f($\Delta F_X$) préalablement déterminée pour lesdites eaux usées exprimant le pourcentage d'abattement de l'au moins un micropolluant ou de l'ensemble des micropolluants à éliminer en fonction du pourcentage de diminution de l'intensité de fluorescence calculée d'un ou plusieurs indicateurs caractéristiques $F_X$,

(iv) réguler la quantité de l'au moins un réactif injectée par le système d'injection dans l'unité de traitement entre ladite entrée et ladite sortie en fonction du pourcentage d'abattement déterminé Ab%,

le système de gestion (14) étant en outre configuré pour :

1) construire une base de données contenant :

- des valeurs de pourcentages d'abattement du ou des micropolluants à éliminer calculées à partir de concentrations du ou des micropolluants mesurées pour une pluralité d'échantillons d'eaux usées traitées dans l'unité de traitement entre ladite entrée et ladite sortie, et
- des valeurs de pourcentages de diminution des intensités de fluorescence mesurées, pour les mêmes échantillons d'eaux usées traitées dans la même unité de traitement entre la même entrée et la même sortie, par spectroscopie de fluorescence 3D pour une pluralité de couples d'une longueur d'onde d'excitation λx_ex et d'une longueur d'onde d'émission λx_em, puis

2) déterminer au moins une corrélation Ab%=f($\Delta F_X$), cette détermination comprenant :

(i) la détermination d'une pluralité de corrélations Ab%=f($\Delta F_X$) au moyen d'un modèle mathématique à partir de ladite base de données, et
(ii) le classement de la pluralité de corrélations déterminées par ordre décroissant de précision de détermination du pourcentage d'abattement, la première corrélation correspondant à une corrélation optimale donnant une valeur d'abattement la plus proche des valeurs de pourcentages d'abattement mesurées pour l'au moins un micropolluant à éliminer et/ou pour un ensemble des micropolluants à éliminer.

## Patentansprüche

1. Steuerungsverfahren zur Entfernung von Mikroschadstoffen aus Abwasser in einer Behandlungseinheit, in welcher die Mikroschadstoffe durch Inkontaktbringen des Abwassers mit mindestens einem Reagens in mindestens einem Reaktor entfernt werden, umfassend die folgenden Schritte:

(a) Bestimmen, zwischen einem Einlass eines der Reaktoren der Behandlungseinheit und einem Auslass desselben Reaktors oder eines stromabwärts der Behandlungseinheit befindlichen Reaktors, mindestens eines Fluoreszenzindikators $F_X$, der repräsentativ für den Abbau eines oder mehrerer der zu entfernenden Mikroschadstoffe abhängig von der Behandlungseinheit ist, wobei jeder Indikator $F_X$ einer emittierten Fluoreszenzintensität für ein Paar einer Erregungswellenlänge λx_ex und einer Emissionswellenlänge λx_em entspricht,
(b) Messen, durch 3D-Fluoreszenzspektroskopie des durch Einlass eintretenden Abwassers, einer ersten Fluoreszenzintensität $F_{X\_in}$, die für den mindestens einen Indikator $F_X$ emittiert wird,
(c) Messen, durch 3D-Fluoreszenzspektroskopie des durch besagten Auslass austretenden Abwassers, einer zweiten Fluoreszenzintensität $F_{X\_out}$, die für den mindestens einen Indikator $F_X$ emittiert wird,
(d) Berechnen eines Rückgangprozentsatzes der Fluoreszenzintensität $\Delta F_X$ zwischen dem Einlass und dem Auslass für jeden Indikator $F_X$ anhand der zuvor gemessenen ersten und zweiten Fluoreszenzintensität: $\Delta F_X = ((F_{X\_in}-F_{X\_out})/F_{X\_in}).100$,
(e) Bestimmen eines Abbauprozentsatzes Ab% zwischen dem Einlass und dem Auslass des mindestens einen zu entfernenden Mikroschadstoffs oder einer Gesamtheit der im Abwasser vorhandenen zu entfernenden Mikroschadstoffe anhand einer zuvor für das Abwasser bestimmten Korrelation Ab%=f($\Delta F_X$), die den Abbauprozentsatz des mindestens einen zu entfernenden Mikroschadstoffs oder der Gesamtheit der zu entfernenden Mikroschadstoffe abhängig von dem berechneten Rückgangprozentsatz der Fluoreszenzintensität eines oder mehrerer charakteristischer Indikatoren $F_X$ ausdrückt,
(f) Regulieren der Menge des mindestens einen in die Behandlungseinheit zwischen dem Einlass und dem Auslass injizierten Reagens abhängig von dem in Schritt (e) bestimmten Abbauprozentsatz Ab%,

das Verfahren umfassend:

1) einen vorherigen Schritt einer Aufbaus einer Datenbank, die Folgendes enthält:

- Werte von Abbauprozentsätzen des oder der zu entfernenden Mikroschadstoffe, berechnet anhand der Konzentrationen des oder der Mikroschadstoffe, die für eine Vielzahl von Abwasserproben gemessen werden, die in der Behandlungseinheit zwischen dem Einlass und dem Auslass behandelt werden, und
- Werte von Rückgangprozentsätzen der gemessenen Fluoreszenzintensitäten, für dieselben in derselben Behandlungseinheit zwischen demselben Einlass und demselben Auslass behandelten Abwasserproben, mittels 3D-Fluoreszenzspektroskopie für eine Vielzahl von Paaren einer Erregungswellenlänge $\lambda x\_ex$ und einer Emissionswellenlänge $\lambda x\_em$, und

2) einen vorherigen Bestimmungsschritts mindestens einer Korrelation Ab%=$f(\Delta F_X)$, wobei dieser Schritt nach dem Schritt eines Aufbauens einer Datenbank durchgeführt wird, und umfassend:

(i) Bestimmen einer Vielzahl von Korrelationen Ab%=$f(\Delta F_x)$ mittels eines mathematischen Modells anhand der aufgebauten Datenbank,
(ii) Ordnen der Vielzahl der bestimmten Korrelationen in absteigender Reihenfolge der Genauigkeit der Bestimmung des Abbauprozentsatzes, wobei die erste Korrelation einer optimalen Korrelation entspricht, die einen Abbauwert ergibt, der den gemessenen Werten der Abbauprozentsätze für den mindestens einen zu entfernenden Mikroschadstoff und/oder für eine Gesamtheit der zu entfernenden Mikroschadstoffe am nächsten kommt.

2. Steuerungsverfahren nach Anspruch 1, wobei die Behandlungseinheit mindestens zwei in Reihe und/oder parallel montierte Reaktoren umfasst und die Schritte (a) bis (e) wie folgt durchgeführt werden:

(i) an dem Einlass und an dem Auslass von mindestens einem Reaktor der Behandlungseinheit, oder
(ii) an dem Einlass des am weitesten stromaufwärtigen Reaktors und an dem Auslass des am weitesten stromabwärtigen Reaktors, oder
(iii) sowohl an dem Einlass und an dem Auslass von mindestens einem Reaktor der Behandlungseinheit als auch an dem Einlass des am weitesten stromaufwärtigen Reaktors und an dem Auslass des am weitesten stromabwärtigen Reaktors, und wobei während des Schritts (f) die Menge des mindestens einen in die Behandlungseinheit zwischen dem Einlass und dem Auslass injizierten Reagens abhängig von jedem der bestimmten Abbauprozentsätze Ab% reguliert wird.

3. Steuerungsverfahren nach Anspruch 1 oder Anspruch 2, wobei Schritt (a) Folgendes umfasst:
Bestimmen als Fluoreszenzindikator $F_x$, der repräsentativ für den Abbau eines oder mehrerer der zu entfernenden Mikroschadstoffe ist, der emittierten Fluoreszenzintensität für das oder die Paare einer Erregungswellenlänge $\lambda x\_ex$ und einer Emissionswellenlänge $\lambda x\_em$, die zumindest in der optimalen Korrelation, optional in den ersten n Korrelationen, verwendet wird bzw. werden, wobei n eine von Null verschiedene ganze Zahl ist.

4. Steuerungsverfahren nach einem der Ansprüche 1 bis 3, wobei während des Schritts (e) die zur Bestimmung des Abbauprozentsatzes verwendete Korrelation Ab%=$f(\Delta F_X)$ die optimale Korrelation, optional eine Korrelation niedrigeren Rangs, ist.

5. Steuerungsverfahren nach Anspruch 4, umfassend den folgenden zusätzlichen Schritt (e'):

(i) Bestimmen einer Abweichung zwischen dem bestimmten Abbauprozentsatz und dem laufenden Abbauprozentsatz und Vergleichen dieser Abweichung mit einem Schwellenwert,
(ii) Verwenden des somit bestimmten Abbauprozentsatzes in Schritt (f), wenn die Abweichung kleiner ist als der Schwellenwert,
(iv) Bestimmen eines anderen Werts des Abbauprozentsatzes, wenn die Abweichung größer ist als der Schwellenwert, und Verwenden dieses anderen Werts des Abbauprozentsatzes in Schritt (f), wobei der andere Wert des Abbauprozentsatzes anhand einer anderen zuvor bestimmten Korrelation Ab%=$f(\Delta F_X)$ bestimmt wird.

6. Steuerungsverfahren nach einem der Ansprüche 1 bis 5, umfassend den folgenden zusätzlichen Schritt:

(i) Messen an einem Zeitpunkt t von Konzentrationen des mindestens einen Mikroschadstoffs in dem zwischen dem Einlass und dem Auslass zu behandelnden Wasser,
(ii) Berechnen der Werte von Abbauprozentsätzen zwischen dem Einlass und dem Auslass des mindestens einen Mikroschadstoffs anhand der gemessenen Konzentrationen,
(iii) Berechnen der Rückgangprozentsätze der Fluoreszenzintensitäten anhand der während der Schritte (b) und (c) an demselben Zeitpunkt t gemessenen ersten und zweiten Fluoreszenzintensität, und

(v) Einspeisen der zu diesem Zeitpunkt t berechneten Abbauwerte und Rückgangprozentsätze der Fluoreszenzintensitäten in die Datenbank.

7. Steuerungsverfahren nach einem der Ansprüche 1 bis 6, wobei, während das mindestens eine Reagens kontinuierlich in die Behandlungseinheit injiziert wird, Schritt (f) eines Regulierens (i) ein Verringern der Menge des injizierten mindestens einen Reagens, wenn der für einen Mikroschadstoff oder für eine Gesamtheit von Mikroschadstoffen bestimmte Abbauprozentsatz größer ist als ein vorbestimmter Schwellenwert, oder (ii) ein Erhöhen der Menge des injizierten mindestens einen Reagens, wenn der für einen Mikroschadstoff oder für eine Gesamtheit von Mikroschadstoffen bestimmte Abbauprozentsatz kleiner ist als ein vorbestimmter Schwellenwert, oder (iii) ein Beibehalten der Menge des injizierten mindestens einen Reagens, wenn der für einen Mikroschadstoff oder für eine Gesamtheit von Mikroschadstoffen bestimmte Abbauprozentsatz größer ist als ein vorbestimmter Schwellenwert, umfasst.

8. Steuerungsverfahren nach einem der Ansprüche 1 bis 7, wobei, während das mindestens eine Reagens diskontinuierlich in die Behandlungseinheit injiziert wird, Schritt (f) eines Regulierens (i) keine Injektion des mindestens einen Reagens, wenn der für einen Mikroschadstoff oder für eine Gesamtheit von Mikroschadstoffen bestimmte Abbauprozentsatz größer ist als ein vorbestimmter Schwellenwert, oder (ii) eine Injektion einer vorbestimmten Menge des mindestens einen Reagens, wenn der für einen Mikroschadstoff oder für eine Gesamtheit von Mikroschadstoffen bestimmte Abbauprozentsatz kleiner ist als ein vorbestimmter Schwellenwert, und ein Austragen derselben Menge des mindestens einen verbrauchten Reagens aus der Behandlungseinheit umfasst.

9. Steuerungsverfahren nach einem der Ansprüche 1 bis 8, wobei die Schritte (b) und (c) mittels mindestens einer mit einem Managementsystem verbundenen Fluoreszenzmesssonde durchgeführt werden.

10. Steuerungssystem (10) zur Entfernung von mindestens einem Mikroschadstoff aus Abwasser einer Behandlungseinheit (1), die mindestens einen Reaktor umfasst, wobei jeder Reaktor einen Einlass (1a) für zu behandelndes Abwasser und einen Auslass (1 b) für behandeltes Abwasser aufweist, das System umfassend:

- mindestens eine Messsonde (11, 12) für 3D-Fluoreszenzspektroskopie, die fluidisch (i) mit dem Einlass eines der Reaktoren der Behandlungseinheit, (ii) mit dem Auslass desselben Reaktors oder eines stromabwärts von der Behandlungseinheit befindlichen Reaktors, oder (iii) sowohl mit dem Einlass als auch mit dem Auslass über ein Verteilerorgan verbunden ist,
wobei die mindestens eine Messsonde (11, 12) zu Folgendem konfiguriert ist: (i) Bestimmen, für mindestens einen Fluoreszenzindikator $F_x$, der repräsentativ für den Abbau eines oder mehrerer der zu entfernenden Mikroschadstoffe abhängig von der Behandlungseinheit ist, wobei jeder Indikator $F_x$ einer emittierten Fluoreszenzintensität für ein Paar einer Erregungswellenlänge $\lambda x\_ex$ und einer Emissionswellenlänge $\lambda x\_em$ entspricht, einer ersten Fluoreszenzintensität $F_x\_in$, die von dem durch den Einlass eintretenden Abwasser emittiert wird, und einer zweiten Fluoreszenzintensität $F_x\_out$, die von dem durch den Auslass austretenden Abwasser emittiert wird,

- ein Injektionssystem (13) für mindestens ein Reagens in die Behandlungseinheit (1),
- ein Managementsystem (14), das mit besagter mindestens einen Messsonde (11, 12) und dem Injektionssystem (13) verbunden ist, und zu Folgendem konfiguriert ist:

(ii) Berechnen eines Rückgangprozentsatzes der Fluoreszenzintensität $\Delta F_x$ zwischen dem Einlass und dem Auslass für jeden Indikator $F_x$ anhand der zuvor gemessenen ersten und zweiten Fluoreszenzintensität $\Delta F_x = ((F_x\_in - F_x\_out)/Fx\_in).1\,00$,
(iii) Bestimmen, zwischen dem Einlass und dem Auslass, eines Abbauprozentsatzes Ab% des mindestens einen zu entfernenden Mikroschadstoffs oder einer Gesamtheit der in dem Abwasser vorhandenen zu entfernenden Mikroschadstoffe anhand einer zuvor für das Abwasser bestimmten Korrelation Ab% $=f(\Delta F_x)$, die den Abbauprozentsatz des mindestens einen zu entfernenden Mikroschadstoffs oder der Gesamtheit der zu entfernenden Mikroschadstoffe abhängig von dem berechneten prozentualen Rückgang der Fluoreszenzintensität eines oder mehrerer charakteristischer Indikatoren $F_x$ ausdrückt,
(iv) Regulieren der Menge des mindestens einen durch das Injektionssystem in die Behandlungseinheit zwischen dem Einlass und dem Auslass injizierten Reagens abhängig von dem bestimmten Abbauprozentsatz Ab%,

wobei das Managementsystem (14) ferner zu Folgendem konfiguriert ist:

1) Aufbauen einer Datenbank, die Folgendes enthält:

- Werte von Abbauprozentsätzen des oder der zu entfernenden Mikroschadstoffe, berechnet anhand Konzentrationen des oder der Mikroschadstoffe, die für eine Vielzahl von Abwasserproben gemessen werden, die in der Behandlungseinheit zwischen dem Einlass und dem Auslass behandelt werden, und
- Werte von Rückgangprozentsätzen der gemessenen Fluoreszenzintensitäten, für dieselben in derselben Behandlungseinheit zwischen demselben Einlass und demselben Auslass behandelten Abwasserproben, mittels 3D-Fluoreszenzspektroskopie für eine Vielzahl von Paaren einer Erregungswellenlänge $\lambda x\_ex$ und einer Emissionswellenlänge $\lambda x\_em$, und anschließend

2) Bestimmen mindestens einer Korrelation Ab%=$f(\Delta F_X)$, diese Bestimmung umfassend:

(i) das Bestimmen einer Vielzahl von Korrelationen Ab%=$f(\Delta F_x)$ mittels eines mathematischen Modells anhand der Datenbank, und
(ii) das Ordnen der Vielzahl der bestimmten Korrelationen in absteigender Reihenfolge der Genauigkeit der Bestimmung des Abbauprozentsatzes, wobei die erste Korrelation einer optimalen Korrelation entspricht, die einen Abbauwert ergibt, der den gemessenen Werten der Abbauprozentsätze für den mindestens einen zu entfernenden Mikroschadstoff und/oder für eine Gesamtheit der zu entfernenden Mikroschadstoffe am nächsten kommt.

**Claims**

1. A method for regulating the removal of micropollutants from wastewater in a treatment unit wherein the micropollutants are removed by bringing the wastewater into contact with at least one reagent in at least one reactor, comprising the following steps:

(a) determining, between an inlet of one of the reactors of the treatment unit and an outlet of the same reactor or of a reactor located downstream of the treatment unit, at least one fluorescence indicator $F_x$ representative of the removal of one or more of the micropollutants to be removed as a function of the treatment unit, each indicator $F_x$ corresponding to a fluorescence intensity emitted for a pair of an excitation wavelength $\lambda x\_ex$ and an emission wavelength $\lambda x\_em$,
(b) measuring by 3D fluorescence spectroscopy of the wastewater entering through said inlet a first fluorescence intensity $F_{x\_in}$ emitted for the at least one indicator $F_x$,
(c) measuring by 3D fluorescence spectroscopy of the wastewater exiting through said outlet a second fluorescence intensity $F_{x\_out}$ emitted for the at least one indicator $F_x$,
(d) calculating a percentage decrease in fluorescence intensity $\Delta F_x$ between said inlet and said outlet for each indicator $F_x$ from the first and second fluorescence intensities previously measured: $\Delta F_x = ((F_{x\_in} - F_{x\_out})/F_{x\_in}).100$,
(e) determining a removal percentage Ab% between said inlet and said outlet of the at least one micropollutant or of a set of micropollutants to be removed present in the wastewater from a correlation Ab%=$f(LIF_x)$ previously determined for said wastewater expressing the removal percentage of the at least one micropollutant or of the set of micropollutants to be removed as a function of the percentage decrease in the calculated fluorescence intensity of one or more characteristic indicators $F_x$,
(f) regulating the amount of the at least one reagent injected into the treatment unit between said inlet and said outlet as a function of the removal percentage Ab% determined in step (e),

the method comprising:

1) a preliminary step of constructing a database containing:

- values of removal percentages of the micropollutant(s) to be removed calculated from concentrations of the micropollutant(s) measured for a plurality of wastewater samples treated in the treatment unit between said inlet and said outlet, and
- values of percentage decreases in measured fluorescence intensities, for the same wastewater samples treated in the same treatment unit between the same inlet and the same outlet, by 3D fluorescence spectroscopy for a plurality of pairs of an excitation wavelength $\lambda x\_ex$ and an emission wavelength $\lambda x\_em$, and

2) a preliminary step of determining at least one correlation Ab%=f($\Delta F_X$), this step being implemented after said step of constructing a database, and comprising:

(i) determining a plurality of correlations Ab%=f($AF_x$) by means of a mathematical model from said constructed database,
(ii) ranking the plurality of determined correlations in descending order of precision of determination of the removal percentage, the first correlation corresponding to an optimal correlation giving a removal value closest to the measured removal percentage values for the at least one micropollutant to be removed and/or for a set of micropollutants to be removed.

2. The method for regulating according to claim 1, wherein the treatment unit comprises at least two reactors mounted in series and/or in parallel and steps (a) to (e) are implemented:

(i) at the inlet and outlet of at least one reactor of the treatment unit, or
(ii) at the inlet of the most upstream reactor and at the outlet of the most downstream reactor, or,
(iii) both at the inlet and outlet of at least one reactor of the treatment unit and at the inlet of the most upstream reactor and at the outlet of the most downstream reactor, and during step (f), the amount of the at least one reagent injected into the treatment unit between said inlet and said outlet is regulated as a function of each of the determined removal percentages Ab%.

3. The method for regulating according to claim 1 or claim 2, wherein, step (a) comprises:
determining as fluorescence indicator $F_x$ representative of the removal of one or more of the micropollutants to be removed, the fluorescence intensity emitted for the wavelength pair(s) of an excitation wavelength $\lambda x\_ex$ and an emission wavelength $\lambda x\_em$ used at least in the optimal correlation, optionally in the first n correlations, n being a non-zero integer.

4. The method for regulating according to any one of claims 1 to 3, wherein during step (e), the correlation Ab%=f($\Delta F_x$) used to determine the removal percentage is the optimal correlation, optionally a lower-ranked correlation.

5. The method for regulating according to claim 4, comprising the following additional step (e'):

(i) determining a variation between the determined removal percentage and the current removal percentage and comparing this variation with a threshold value,
(ii) using the removal percentage thus determined in step (f) if the variation is lower than the threshold value,
(iii) determining another removal percentage value if the variation is greater than the threshold value and using this other removal percentage value in step (f), the other removal percentage value being determined from another correlation Ab%=f($LIF_x$) previously determined.

6. The method for regulating according to any one of claims 1 to 5, comprising the following additional step:

(i) measuring at an instant t concentrations of the at least one micropollutant in the water to be treated between said inlet and said outlet,
(ii) calculating the removal percentage values between said inlet and said outlet of the at least one micropollutant from the measured concentrations,
(iii) calculating the percentage decreases in fluorescence intensities from the first and second fluorescence intensities measured during steps (b) and (c) at the same instant t, and
(iv) injecting into the database the removal values and the percentage decreases in fluorescence intensities calculated at this instant t.

7. The method for regulating according to any one of claims 1 to 6, wherein, the at least one reagent being injected continuously into the treatment unit, regulation step (f) comprises (i) a reduction in the amount of the at least one reagent injected when the removal percentage determined for a micropollutant or for a set of micropollutants is greater than a predefined threshold value, or (ii) an increase in the amount of the at least one reagent injected when the removal percentage determined for a micropollutant or for a set of micropollutants is lower than a predefined threshold value, or (iii) maintaining the amount of the at least one reagent injected when the removal percentage determined for a micropollutant or for a set of micropollutants is greater than a predefined threshold value.

8. The method for regulating according to any one of claims 1 to 7, wherein, the at least one reagent being injected discontinuously into the treatment unit, regulation step (f) comprises (i) no injection of the at least one reagent when the removal percentage determined for a micropollutant or for a set of micropollutants is greater than a predefined threshold value or (ii) an injection of a predefined amount of the at least one reagent when the removal percentage determined for a micropollutant or for a set of micropollutants is lower than a predefined threshold value and the extraction of the same amount of the spent at least one reagent out of the treatment unit.

9. The method for regulating according to any one of claims 1 to 8, wherein steps (b) and (c) are carried out by means of at least one fluorescence measurement probe connected to a management system.

10. A system for regulation (10) of the removal of at least one micropollutant from the wastewater of a treatment unit (1) comprising at least one reactor, each reactor having an inlet (1a) of the wastewater to be treated and an outlet (1b) of the treated wastewater, the system comprising:

- at least one measurement probe (11, 12) by 3D fluorescence spectroscopy, fluidically connected (i) to the inlet of one of the reactors of the treatment unit, (ii) to the outlet of the same reactor or of a reactor located downstream of the treatment unit, or (iii) both to said inlet and to said outlet via a distribution member, said at least one measurement probe (11, 12) being configured to: (i) determine for at least one fluorescence indicator $F_x$ representative of the removal of one or more of the micropollutants to be removed as a function of the treatment unit, each indicator $F_x$ corresponding to a fluorescence intensity emitted for a pair of an excitation wavelength $\lambda x\_ex$ and an emission wavelength $\lambda x\_em$, a first fluorescence intensity $F_x\_in$ emitted by the wastewater entering through said inlet and a second fluorescence intensity $F_x\_out$ emitted by the wastewater exiting through said outlet,
- an injection system (13) of at least one reagent into the treatment unit (1),
- a management system (14) connected to said at least one measurement probe (11, 12) and to the injection system (13), configured to:

(ii) calculate a percentage decrease in fluorescence intensity $\Delta F_x$ between said inlet and said outlet for each indicator $F_x$ from the first and second fluorescence intensities previously measured $\Delta F_x = ((F_x\_in - F_x\_out)/F_x\_in).100$,
(iii) determine between said inlet and said outlet a removal percentage Ab% of the at least one micropollutant or of a set of micropollutants to be removed present in the wastewater from a correlation Ab%=f(LIF$_x$) previously determined for said wastewater expressing the removal percentage of the at least one micropollutant or of the set of micropollutants to be removed as a function of the percentage decrease in the calculated fluorescence intensity of one or more characteristic indicators $F_x$,
(iv) regulate the amount of the at least one reagent injected by the injection system into the treatment unit between said inlet and said outlet as a function of the determined removal percentage Ab%,

the management system (14) being further configured to:

1) construct a database containing:

- values of removal percentages of the micropollutant(s) to be removed calculated from concentrations of the micropollutant(s) measured for a plurality of wastewater samples treated in the treatment unit between said inlet and said outlet, and
- values of percentage decreases in measured fluorescence intensities, for the same wastewater samples treated in the same unit treatment between the same inlet and the same outlet, by 3D fluorescence spectroscopy for a plurality of pairs of an excitation wavelength $\lambda x\_ex$ and an emission wavelength $\lambda x\_em$, then

2) determine at least one correlation Ab%=f($\Delta F_x$), this determination comprising:

(i) the determination of a plurality of correlations Ab%=f(AF$_x$) by means of a mathematical model from said database, and
(ii) the ranking of the plurality of determined correlations in descending order of precision of determination of the removal percentage, the first correlation corresponding to an optimal correlation giving a removal value closest to the measured removal percentage values for the at least one micropollutant to be removed and/or for a set of micropollutants to be removed.

Fig. 1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3009789 A1
- EP 3348995 B1
- EP 3174833 A1
- JP 2002166265 A
- WO 2014186167 A1

**Littérature non-brevet citée dans la description**

- **PARK et al.** *Chemosphere*, 2018, vol. 193, 530-537
- Comparison of the new Cl2/03/UV process with different ozone- and UV-based AOPs for wastewater treatment at pilot scale: Removal of pharmaceuticals and changes in fluorescing organic matter. **SGROI MASSIMILIANO et al.** SCIENCE OF THE TOTAL ENVIRONMENT. ELSEVIER, 06 October 2020, vol. 765
- Development of fluorescence surrogates to predict the ferrate (VI) oxidation of pharmaceuticals in wastewater effluents. **NIE JIANXIN et al.** WATER RESEARCH. ELSEVIER, 31 July 2020, vol. 185
- Fluorescence excitation/emission matrices as a tool to monitor the removal of organic micropollutants from wastewater effluents by adsorption onto activated carbon. **GUILLOSSOU et al.** WATER RESEARCH. ELSEVIER, 14 December 2020, vol. 190
- **LAWAETZ, A.J.** ; **STEDMON, C.A**. Fluorescence intensity calibration using the raman scatter peak of water. *Appl. Spectrosc.*, 2009, vol. 63, 936-940